# FASCICULE DE BREVET EUROPEEN

(11) **EP 3 672 607 B1**
(45) Date de publication et mention de la délivrance du brevet: **26.10.2022**
(21) Numéro de dépôt: 18758620.1
(22) Date de dépôt: 23.08.2018
(51) Int. Cl.: A61K 35/51, C12M 3/00, C12N 5/073, B33Y 70/00, B33Y 10/00, A61P 17/02, A61P 27/02

(54) **COMPOSITION COMPRENANT DE LA GELEE DE WHARTON, PROCEDE DE PREPARATION ET UTILISATIONS**
ZUSAMMENSETZUNG MIT WHARTON-SULZE, VERFAHREN ZU IHRER HERSTELLUNG UND IHRE VERWENDUNG
COMPOSITION COMPRISING WHARTON'S JELLY, METHOD FOR PREPARING SAME AND USES THEREOF

(30) Priorité: 23.08.2017 FR 1757828
(43) Date de publication de la demande: 01.07.2020
(73) Titulaire: TBF Genie Tissulaire (TBF), 69780 Mions (FR)
(72) Inventeur: BARNOUIN, Laurence, 38460 Venerieu (FR)
(74) Mandataire: Tripoz, Inès
(86) Numéro de dépôt international: PCT/EP2018/072829
(87) Numéro de publication internationale: WO 2019/038411

(56) Documents cités:
- WO-A1-2014/039429
- WO-A1-2015/066705
- WO-A1-2017/140914
- FR-A1- 2 949 042
- KR-A- 20140 091 801
- US-A1- 2008 181 967
- EL-SERAFI AHMED T ET AL: "Skin regeneration in three dimensions, current status, challenges and opportunities", DIFFERENTIATION, vol. 96, 15 juin 2017 (2017-06-15), pages 26-29, XP085258996, ISSN: 0301-4681, DOI: 10.1016/J.DIFF.2017.06.002
- LAURENCE BARNOUIN (ORATEUR) 1 ET AL: "Produit d'ingénierie tissulaire comme chambre de régénération nerveuse - vaisseau ombilical traité, déshydraté et stérile", HAND SURGERY AND REHABILITATION,, vol. 36, no. 6, 1 décembre 2017 (2017-12-01), page 486, XP009503710, ISSN: 2468-1229

## Description

La présente invention est définie dans les revendications. La présente invention concerne un biomatériau consistant en un broyat de gelée de Wharton viro-inactivée et qui peut être lyophilisé, et son procédé de production. L'invention concerne également une composition comprenant au moins ledit biomatériau, qui peut être lyophilisée ainsi que son procédé de production. La composition selon la présente invention est destinée à être utilisée dans la cicatrisation tissulaire. Lors d'un traumatisme des tissus biologiques, que celui-ci soit causé par une infection, une altération mécanique ou une pathologie, les cellules composant lesdits tissus sont détruites et la matrice extracellulaire est désorganisée. Cette altération des tissus induit un processus biologique de cicatrisation visant à réparer la ou les lésion(s) tissulaire(s). Plusieurs applications visant à améliorer le processus de cicatrisation ont été développées, notamment par introduction de compositions *in situ* au site lésé. Ces applications visent également la libération locale de principes actifs destinés à favoriser la cicatrisation. Dans un mode de réalisation particulier, la composition est utilisée dans le traitement d'une lésion cutanée ou un traitement de lésion de la surface oculaire. Dans un autre mode de réalisation, la composition est utilisée dans le comblement osseux, ou pour le comblement d'une alvéole dentaire. La présente invention concerne également l'utilisation de la composition comme facteur d'enrobage dans la bio-impression.

Lors d'un traumatisme de l'épiderme, ou du derme et de l'épiderme, peuvent apparaitre une ou des lésions telles qu'une plaie cutanée, une plaie de cicatrisation, une brûlure ou inflammation aigue ou chronique allergique comme l'eczéma. Il existe également des plaies chroniques, causées notamment par un ulcère variqueux ou un ulcère diabétique. De nombreuses compositions destinées à améliorer le processus de cicatrisation sont connues, notamment sous formes de gels, de pommades ou de pansements ou bandages comprenant des principes actifs libérés lors de leur utilisation.

Lors des lésions de la surface oculaire, ou lors d'une diminution de la sécrétion lacrymale dans le cadre de différentes pathologies comme le syndrome de l'œil sec, des infections ou inflammations entrainent une irritation de la conjonctive. Le syndrome de l'œil sec est une pathologie chronique caractérisée en ce que les malades ne produisent pas assez de larmes pour assurer l'hydratation continue de leurs yeux. De plus, cette pathologie induit une irritation pouvant conduire à une vive douleur, une sensibilité à la lumière ou une sensation de démangeaison chez les malades. Plusieurs compositions existent, notamment sous forme de collyres, afin de rétablir le film lacrymal et ainsi lutter contre les symptômes de la maladie.

Lors de certaines lésions osseuses, une perte de matière peut survenir, ceci est par exemple le cas lors de certaines opérations chirurgicales où un praticien doit pratiquer une exérèse d'un fragment osseux. Le recours à une greffe de tissu osseux est alors souvent nécessaire afin de procéder à la reconstruction, il existe également des compositions chimiques proches de la phase minérale de l'os qui peuvent être utilisées pour combler les cavités osseuses. Également, lors de la réalisation d'une arthrodèse, le recours à un comblement de l'espace articulaire est nécessaire pour obtenir une fusion. En effet, l'acte chirurgical de l'arthrodèse consiste en une greffe d'un substitut osseux dans l'espace articulaire pour permettre une fusion osseuse ayant pour but de corriger une déformation ou d'obtenir l'indolence d'une articulation lésée.

Lors de la perte d'une ou plusieurs dents, que ce soit à la suite d'un traumatisme, d'une pathologie ou d'une intervention chirurgicale, un phénomène de résorption osseux se produit au niveau de l'alvéole dentaire désormais vacante. Afin de pouvoir procéder à la pose d'un implant dentaire, il convient que le tissu de comblement de l'alvéole dentaire soit solide et sain afin que l'implant soit efficacement fixé.

La gelée de Wharton est un tissu conjonctif gélatineux entourant la veine et les deux artères du cordon ombilical des mammifères. La gelée de Wharton est une substance particulièrement riche en éléments constitutifs de la matrice extracellulaire des tissus conjonctifs, notamment en glycosaminoglycanes et protéoglycanes ; ainsi qu'en fibres de collagène (types I, III, IV et V). La gelée de Wharton comprend également de nombreux facteurs de croissance tels que, notamment, les facteurs de croissance des fibroblastes (FGF), les facteurs de croissance I ressemblant à l'insuline (IGF-I), les facteurs de croissance transformant (TGF), les facteurs de croissance dérivés des plaquettes (PDGF) et les facteurs de croissance épidermique (EGF).

Ces propriétés structurelles de la gelée de Wharton peuvent aussi être utilisées pour leurs propriétés d'aide à la cicatrisation des lésions, notamment des lésions cutanées ou des lésions de la surface oculaire. En effet, ces éléments constitutifs de la gelée de Wharton participent à l'amélioration des processus biologiques de cicatrisation et de réduction de l'inflammation chez un patient.

Cependant, il a été démontré que des virus de type Polyomavirus peuvent infecter les cellules souches mésenchymateuses présentes dans la gelée de Wharton (Cason, C. et al. (2016), "SV40 Infection of Mesenchymal Stromal Cells From Wharton's Jelly Drives the Production of Inflammatory and Tumoral Mediators" Journal of Cellular Physiology. DOI: 10.1002/jcp.25723 et Comar, M. et al. (2014), "In vivo détection of polyomaviruses JCV and SV40 in mesenchymal stem cells from human umbilical cords." Pediatr Blood Cancer, 61: 1347-1349. doi:10.1002/pbc.24943). Il existe donc un risque sanitaire relatif à la contamination d'un patient qui serait traité avec une composition comprenant de la gelée de Wharton dont une viro-inactivation efficace n'aurait pas été assurée.

Différentes stratégies destinées à l'obtention d'un gel pharmaceutique pour application topique, obtenu à partir de la gelée de Wharton, ont été développées. Ces stratégies présentent pour inconvénients techniques une perte de matière biologique, et donc des substances d'intérêt, ou de ne pas permettre une désinfection satisfaisante du tissu biologique.

On peut citer par exemple la demande de brevet WO 2015/179711, déposée au nom de MIMEDX GROUP, dans laquelle est décrit un procédé du traitement de la gelée de Wharton, seule ou en combinaison avec un autre tissu placentaire, et dont le produit est destiné à des applications médicales. Ce procédé est notamment caractérisé en ce que l'étape de désinfection est assurée par un nettoyage en solution hyper-isotonique suivi d'un bain en solution d'antibiotiques. De plus, le broyage de la gelée de Wharton est réalisé par un broyage mécanique ou cryogénique sur une substance déshydratée. Cette étape du broyage implique un échauffement du tissu biologique déshydraté pouvant conduire à la dégradation de celui-ci.

Dans la demande de brevet US 2008/0181967, l'étape de broyage de la gelée de Wharton est réalisée par sonication ou par l'usage d'un mixeur. La méthode qui est divulguée dans cette demande présente pour inconvénient technique que la fraction insoluble est retirée par filtration ou centrifugation, ce qui implique une perte en substances d'intérêt issues du tissu biologique.

De plus, les compositions décrites dans ce document peuvent être obtenues à partir de tissus biologiques qui peuvent être tout ou partie d'un tissu placentaire ou tout ou partie d'un tissu du cordon ombilical, ou une combinaison de ces éléments. Ces tissus biologiques sont placés dans une solution, puis broyés, afin d'obtenir une suspension.

L'invention décrite dans la présente demande ne peut pas contenir tout ou partie d'un tissu placentaire. En effet, ce type de tissu biologique ne permet pas d'obtenir la structure de la composition selon l'invention sous forme de gelée, en ce que les tissus placentaires comprennent notamment des fibres de collagène trop épaisses. De plus, le placenta est un tissu constitué de lacunes et parois vasculaires (villosités et chambres intervilleuses) intervenant dans les échanges vasculaires materno-fœtaux, ce type de tissu, très dense et épais, ne permet pas non plus d'obtenir la structure de la composition sous forme de gelée selon l'invention. La composition selon l'invention ne peut pas être obtenue à partir de tout ou partie d'un tissu placentaire, et ne comprend donc ni fibres de collagène épaisses, ni lacunes et parois vasculaires.

Dans le brevet européen EP 0 310 507, au nom de BONTEMPS, est décrit un procédé de traitement d'une substance fœtale qui peut être la gelée de Wharton, dont le produit obtenu est destiné à un usage topique en cosmétique. Ce procédé est caractérisé notamment par une étape de broyage au robot de coupe (mixeur) de la substance fœtale et de sa filtration. Ces caractéristiques du broyage impliquent que le procédé élimine une partie de la composition des substances d'intérêt, à savoir une fraction qui n'a pas pu être réduite par le mixeur.

La demande de brevet WO 2015/187812, au nom de TISSUETECH, où sont décrites des compositions obtenues à partir de membrane amniotique et de tissu ombilical réduits en morceaux. Les tissus d'intérêt incluent la gelée de Wharton seule ou en combinaison avec la membrane amniotique. Ces compositions sont destinées au traitement de diverses pathologies sous forme notamment de traitements dermiques et oculaires. Le procédé décrit est caractérisé en ce qu'il ne comprend pas d'étape de décellularisation, l'importance de la conservation des cellules souches de la composition étant indiquée. De plus, aucune étape de viro-inactivation n'est décrite dans ce document. En conséquence, cette composition peut comprendre des virus, tant dans le milieu intracellulaire qu'extracellulaire.

Dans le document KR 2014-0091801 est décrite une composition collagénique destinée au revêtement d'un milieu de culture de cellules souches endothéliales issues de vaisseaux sanguins du cordon ombilical. Cette composition collagénique est obtenue par extraction de la fraction collagénique d'un segment de cordon ombilical humain et digestion protéique.

Dans le document FR 2 949 042 est décrit un procédé de traitement d'un tissu de type cartilagineux, caractérisé notamment en au moins une étape de lavage à l'éthanol 70% et au moins une étape de désinfection au peroxyde d'hydrogène. Ce procédé est appliqué à un tissu conjonctif souple mais résistant qu'est le cartilage, et non à un tissu biologique mou gélatineux tel que la gelée de Wharton.

Dans le document WO 2015/066705 est décrit un procédé de préparation d'une bio-encre, obtenue à partir de la fraction collagénique de tissus tendineux, et destinée à la réparation de tissus biologiques défectueux. Ce produit présente comme inconvénient de fournir une matrice biologique de support uniquement composé de collagène, et dont la viro-inactivation est insatisfaisante dans le cadre de l'implantation du tissu bio-imprimé chez un patient.

Dans le document El-Serafi et al. (2017). Skin Régénération in Three Dimensions, Current Status, Challenges and Opportunities. Differentiation. Vol 96. Pages 26-29 DOI 10.1016/j.diff.2017.06.002. est indiqué qu'une matrice consistant en du collagène, du fibrogène et de la thrombine est particulièrement appréciée dans l'ingénierie tissulaire de tissu cutané en bio-impression. Ce produit présente comme inconvénient de ne fournir un contexte biologique de support uniquement composé de collagène, et dont la viro-inactivation est insatisfaisante dans le cadre de l'implantation du tissu bio-imprimé chez un patient.

Dans le document WO 2014/039429 sont décrites des matrices tridimensionnelles destinées à la bio-impression, obtenues à partir de tissus placentaires, mais dont la stérilité n'est pas satisfaisante au regard des exigences de la culture cellulaire.

Les compositions biologiques destinées à être appliquées sur un tissu lésé, tel que par exemples une lésion ou plaie cutanée ; ou une zone irritée telle que les yeux dans la pathologie du syndrome de l'œil sec, présentent un risque de transmission d'une infection virale. L'origine virale peut être du donneur lui-même, d'un risque de contamination lors de la collecte du tissu à l'accouchement, lors des traitements préparatoires de tissu, ou lors du transport de ce tissu. De fait, un procédé efficace tant de désinfection que de conservation de ces tissus est vital pour la sécurité des patients.

Toutes ces compositions présentent certains inconvénients et notamment des problèmes de compatibilité biologique, de conditionnement, de conservation et de mise à disposition des praticiens, de perte de substances d'intérêt thérapeutique, et potentiellement de viro-inactivation. Soit que le traitement n'est pas suffisamment efficace, soit qu'il est trop long et/ou destructeur vis-à-vis du matériau naturel.

Également, des modes de production de compositions décrites dans l'art antérieur, incluant des tissus issus de placenta, ne permettent pas d'obtenir une structure propice à une mise en forme simple et une utilisation facilitée.

Un objectif de la présente invention est donc de proposer un biomatériau consistant en un broyat de gelée de Wharton, et son procédé de préparation, permettant de résoudre les problèmes présentés par les compositions et leurs procédés d'obtention ci-dessus décrits.

La présente invention consiste en un biomatériau consistant en un broyat homogène de gelée de Wharton viro-inactivée caractérisé en ce que les virus ont perdu leurs capacités pathogéniques et de réplication par une diminution de leur population de 4 log lors des titrages résiduels qui suivent une ou deux étapes chimiques indépendantes et en ce que le broyat ne comprend aucun fragment ou morceau dont les dimensions sont significativement supérieures à celles des éléments majoritaires du broyat et qu'il comprend l'intégralité des éléments constitutifs du tissu biologique initial qui est la gelée de Wharton, et qui peut être lyophilisé. Le broyat de gelée de Wharton présente pour caractéristique d'être homogène, et d'inclure toutes les substances d'intérêt du tissu biologique dont il est issu.

La présente invention consiste en un biomatériau consistant en un broyat homogène de gelée de Wharton viro-inactivée caractérisé en ce que les virus ont perdu leurs capacités pathogéniques et de réplication par une diminution de leur population de 4 log lors des titrages résiduels qui suivent une ou deux étapes chimiques indépendantes et en ce que le broyat ne comprend aucun fragment ou morceau dont les dimensions sont significativement supérieures à celles des éléments majoritaires du broyat et qu'il comprend l'intégralité des éléments constitutifs du tissu biologique initial qui est la gelée de Wharton, et qui peut être lyophilisé. Ledit biomatériau est dépourvu de produit ou sous-produit issu de placenta.

La présente invention consiste en un biomatériau consistant en un broyat homogène de gelée de Wharton viro-inactivée caractérisé en ce que les virus ont perdu leurs capacités pathogéniques et de réplication par une diminution de leur population de 4 log lors des titrages résiduels qui suivent une ou deux étapes chimiques indépendantes et en ce que le broyat ne comprend aucun fragment ou morceau dont les dimensions sont significativement supérieures à celles des éléments majoritaires du broyat et qu'il comprend l'intégralité des éléments constitutifs du tissu biologique initial qui est la gelée de Wharton, et qui peut être lyophilisé. Ledit biomatériau est dépourvu de fibres de collagène épaisses et/ou de lacunes et parois vasculaires.

Le biomatériau peut être avantageusement distribué dans des contenants caractérisés par des formes précises avant lyophilisation, et ainsi le biomatériau peut être préformé par exemples en disques, cônes ou cubes pour une manipulation aisée dans le site et réhydraté *in situ.*

Lors du traitement de la surface oculaire, le biomatériau déshydraté peut être introduit dans le cul de sac conjonctival et être a posteriori réhydraté.

Le biomatériau peut également être réhydraté avant son application in situ.

La présente invention concerne également un procédé de préparation du biomatériau selon l'invention. Le procédé peut également inclure une étape de lyophilisation.

L'invention concerne également une composition comprenant au moins le biomatériau.

La présente invention concerne également l'utilisation de ladite composition comprenant au moins le biomatériau qui peut être lyophilisée, dans le traitement des lésions tissulaires, notamment des lésions cutanées ou des lésions des surfaces oculaires.

La présente invention concerne également une méthode pour traiter les lésions tissulaires, notamment des lésions cutanées ou les lésions des surfaces oculaires, par administration topique d'une quantité thérapeutiquement suffisante de ladite composition.

La présente invention concerne également l'utilisation de ladite composition comprenant au moins le biomatériau qui peut être lyophilisée, dans le comblement osseux ou le comblement d'une alvéole dentaire.

La présente invention concerne également une méthode de comblement pour traiter les pertes osseuses, notamment par le comblement desdites pertes osseuses, par administration d'une quantité thérapeutiquement suffisante de ladite composition.

La présente invention concerne également une méthode de comblement pour bloquer ou fusionner une articulation lors de la réalisation d'une arthrodèse, par administration d'une quantité thérapeutiquement suffisante de ladite composition.

La présente invention concerne également une méthode de comblement pour remplacer une articulation lors de la réalisation d'une arthroplastie primaire ou secondaire, par administration d'une quantité thérapeutiquement suffisante de ladite composition.

La présente invention concerne également une méthode pour traiter les alvéoles dentaires, notamment par le comblement desdites alvéoles dentaires, par administration d'une quantité thérapeutiquement suffisante de ladite composition.

La demanderesse a également mis en évidence l'intérêt technique de l'utilisation de la composition comme facteur d'enrobage dans la bio-impression. En effet, la composition selon l'invention permet avantageusement d'enrober des biomatériaux ou cellules destinés à la bio-impression. La composition selon l'invention, en plus de son environnement biologique favorable précédemment décrit, permet de favoriser les échanges thermiques. Il est en effet connu que certains matériaux doivent être chauffés et refroidis avant bio-impression, la composition selon l'invention étant stable à la chaleur dans ses caractéristiques, elle est donc un excellent facteur d'enrobage dans la bio-impression.

Définitions utilisées dans la présente demande.

Certaines expressions ou mots peuvent être utilisés dans la présente demande pour indiquer des éléments techniques, faits, propriétés ou caractéristiques. Leurs significations sont données ci-après :

Par « gelée de Wharton », on entend le tissu biologique gélatineux présent dans le cordon ombilical des mammifères dont la veine et les deux artères naturellement incluses au sein dudit tissu biologique gélatineux ont été retirées. Dans la présente invention, le terme « gelée de Wharton » peut être entendu comme comprenant ou non la membrane amniotique entourant la gelée de Wharton du cordon ombilical. Dans la présente invention, le terme « gelée de Wharton » est entendu comme ne comprenant pas de fibres de collagène épaisses et/ou de lacunes et parois vasculaires (villosités et chambres intervilleuses).

Par « segment de gelée de Wharton », on entend une fraction du tissu biologique d'origine, qui est le cordon ombilical, et qui a été sectionné en deux extrémités de telle sorte que la fraction est isolée du tissu biologique d'origine ; la veine et les deux artères ont été retirées. Le « segment de gelée de Wharton » est donc défini par une portion de cordon ombilical, sectionné en ses deux extrémités, constitué de la gelée de Wharton et de la membrane amniotique entourant ladite gelée de Wharton. Comme défini ci-avant, le terme « gelée de Wharton » pouvant comprendre ou non la membrane amniotique entourant la gelée de Wharton du cordon ombilical, le terme « segment de gelée de Wharton » est donc également défini comme une portion de cordon ombilical, sectionné en ses deux extrémités, constitué de la gelée de Wharton seule.

Par « viro-inactivation », on entend une technique qui permet de réduire fortement ou totalement, et définitivement, la capacité d'action des virus. Ceux-ci, définis comme inactivés, perdent leurs capacités pathogéniques et de réplication par une diminution de leur population de 4 log lors des titrages résiduels qui suivent une ou deux étapes chimiques indépendantes, que ce soit sur des virus enveloppés, ou non enveloppés, ADN ou ARN.

Par « lyophilisation », on entend une technique visant à dessécher un produit préalablement surgelé par sublimation. Plus précisément, le liquide à ôter du produit est dans un premier temps transformé en glace par congélation ; puis par une dessiccation primaire, sous vide, la glace est sublimée ; enfin par une dessiccation secondaire, les molécules d'eau à la surface du produit sont extraites par désorption.

Par « alcools », on entend tout composé organique dont l'un des carbones est lié à un groupe hydroxyle (-OH).

Par « peroxydes », on entend tout composé chimique contenant un groupe fonctionnel de formule générale R-O-O-R' (deux atomes d'oxygène voisins liés entre eux) où R et R' sont des atomes d'hydrogène ou des radicaux alkyls quelconques.

Par « broyat », on entend une composition obtenue par une technique de broyage d'un tissu biologique. Dans la présente invention, la technique de broyage est caractérisée en ce qu'elle permet l'obtention d'un broyat homogène. A savoir que l'intégralité du tissu biologique a été réduit par broyage en une composition dont tous les constituants sont de taille identique ou quasi-identique. Cela signifie que dans la présente invention, le « broyat » est caractérisé par son homogénéité, il ne comprend aucun fragment ou morceau dont les dimensions seraient significativement supérieures à celles des éléments majoritaires du broyât. En conséquence, le « broyat » tel que défini dans la présente invention est caractérisé en ce qu'il comprend l'intégralité ou quasi-intégralité des éléments constitutifs du tissu biologique initial qui est la gelée de Wharton. Également, selon la présente invention, le broyat n'est jamais réalisé en solution. L'étape de broyage du segment de gelée de Wharton est réalisée en ce que ledit segment de gelée de Wharton est broyé après lavage, sans adjonction ou suppression de liquide, pour obtenir un gel.

Par « lésion tissulaire », on entend une modification pathologique d'un tissu biologique, induite notamment par une plaie ou blessure ou conséquence d'une réaction infectieuse ou inflammatoire ou brûlure ou ulcère ou geste chirurgical ou traumatique de tout autre dommage tissulaire. A titre d'exemples non limitatifs, le tissu biologique peut être le tissu dermique, le tissu musculaire, le tissu intra-osseux, le tissu intracérébral, le tissu nerveux, le tissu viscéral.

Par « lésion cutanée », on entend une modification pathologique ou traumatologique de l'épiderme, ou du derme et de l'épiderme, induite notamment par une plaie ou blessure ou conséquence d'une réaction infectieuse ou inflammatoire ou brûlure ou ulcère de tout autre dommage tissulaire. A titre d'exemples non limitatifs, les lésions cutanées suivantes peuvent être citées : un eczéma, un herpès, un zona, une brûlure thermique ou chimique, un ulcère variqueux ou diabétique, une coupure ou cicatrisation retardée ou non jointe.

Par « bio-impression », on entend tout procédé d'ingénierie tissulaire utilisant les principes de l'impression 3D appliqués à la matière vivante cellulaire ou de biomatériaux, et procédant ainsi à l'assemblage couche par couche des constituants des tissus biologiques selon des architectures prédéfinies par conception numérique. Les hommes du métier spécialisés dans la bio-impression la définissent comme « *l'utilisation de procédés de fabrication numérique permettant d'organiser et d'assembler en 3D les constituants des tissus biologiques dans le but de produire des greffons pour la médecine régénératrice ou des modèles physiologiques pour la recherche biomédicale »* (F. Guillemot, V. Mironov & M. Nakamura, Biofabrication (2010)).

Par « facteur d'enrobage dans la bio-impression », on entend une matrice dans laquelle les biomatériaux ou cellules destinés à la bio-impression sont compris. Cette matrice enrobe les biomatériaux ou cellules lors de la bio-impression, elle sert tant de support que de protection à ceux-ci. L'ensemble constitué de la matrice et des biomatériaux ou cellules destinés à la bio-impression est communément nommé bioencre.

Par « perte osseuse », on entend toute perte de matière d'un os consécutive par exemples à une pathologie, un traumatisme ou un acte chirurgical.

Par « comblement osseux » on entend notamment l'acte d'introduire au niveau d'un tissu osseux chez un patient un matériau visant à remplacer une perte de matière osseuse chez ledit patient. Ladite perte de matière osseuse peut, par exemples, être consécutive à une trépanation, consécutive à une exérèse d'un fragment d'os, consécutive à une ostéoporose, consécutive au forage d'une cavité dans un os, consécutive à un traumatisme, consécutive à une fracture, consécutive à une tumeur. On entend également par « comblement osseux » l'acte d'ajouter un matériau au contact d'un tissu osseux sans qu'il y ait eu perte de matière osseuse. Par exemples, l'ajout d'un matériau dans le cadre d'un comblement osseux peut être réalisé lors d'un acte d'arthrodèse, ou d'un acte d'arthroplastie primaire ou secondaire.

Par « alvéole dentaire », on entend les cavités présentes dans le maxillaire (supérieur ou inférieur) dans lesquelles viennent se loger les dents. Dans la présente demande, il est entendu que les alvéoles dentaires sont accessibles en ce que les dents normalement associées aux alvéoles dentaires sont absentes. Cette absence de dents des alvéoles dentaires peut être par exemples consécutive à une pathologie, un traumatisme ou un acte chirurgical.

Par « comblement d'une alvéole dentaire », on entend l'acte d'introduire dans une alvéole dentaire chez un patient un matériau visant à combler l'alvéole laissée vacante par l'absence de la dent normalement associée à l'alvéole.

Par « environ », lorsque ce terme est utilisé relativement à une valeur numérique, on entend toute valeur numérique comprise entre plus et moins 10 % de la valeur numérique auquel est associé le terme environ.

Le biomatériau selon l'invention est ci-après décrit. Ledit biomatériau peut également être lyophilisé. La composition comprenant au moins ledit biomatériau est également décrite. Ladite composition peut également être lyophilisée. Ces compositions sont notamment particulièrement riches en collagène de type I et en glycosaminoglycanes sulfatés et non sulfatés.

L'invention concerne ainsi un biomatériau consistant en un broyat homogène de gelée de Wharton viro-inactivée caractérisé en ce que les virus ont perdu leurs capacités pathogéniques et de réplication par une diminution de leur population de 4 log lors des titrages résiduels qui suivent une ou deux étapes chimiques indépendantes et en ce que le broyat ne comprend aucun fragment ou morceau dont les dimensions sont significativement supérieures à celles des éléments majoritaires du broyat et qu'il comprend l'intégralité des éléments constitutifs du tissu biologique initial qui est la gelée de Wharton.

L'invention concerne ainsi un biomatériau consistant en un broyat homogène de gelée de Wharton viro-inactivée caractérisé en ce que les virus ont perdu leurs capacités pathogéniques et de réplication par une diminution de leur population de 4 log lors des titrages résiduels qui suivent une ou deux étapes chimiques indépendantes et en ce que le broyat ne comprend aucun fragment ou morceau dont les dimensions sont significativement supérieures à celles des éléments majoritaires du broyat et qu'il comprend l'intégralité des éléments constitutifs du tissu biologique initial qui est la gelée de Wharton et lyophilisé.

Dans un mode de réalisation, le biomatériau selon l'invention est caractérisé en ce qu'il comprend en outre une teneur massique en acide hyaluronique comprise entre 5 et 14 mg par gramme de composition (5 mg/g ≤ teneur massique en acide hyaluronique ≤ 14 mg/g).

Dans un mode de réalisation, le biomatériau selon l'invention est caractérisé en ce qu'il comprend en outre une teneur massique en acide hyaluronique comprise entre 7 et 12 mg par gramme de composition (7 mg/g ≤ teneur massique en acide hyaluronique ≤ 12 mg/g).

Dans un mode de réalisation, le biomatériau selon l'invention est caractérisé en ce qu'il comprend en outre une teneur massique en facteur de croissance TGF-β1 comprise entre 0,6 et 1,9 ng par gramme de composition (0,6 ng/g ≤ teneur massique en TGF-β1 ≤ 1,9 ng/g).

Dans un mode de réalisation, le biomatériau selon l'invention est caractérisé en ce qu'il comprend en outre une teneur massique en facteur de croissance TGF-β1 comprise entre 0,8 et 1,7 ng par gramme de composition (0,8 ng/g ≤ teneur massique en TGF-β1 ≤ 1,7 ng/g).

L'invention concerne une composition comprenant au moins ledit biomatériau.

Dans un mode de réalisation, l'invention concerne une composition comprenant au moins ledit biomatériau, caractérisée en ce qu'elle est lyophilisée.

Dans un mode de réalisation, la composition selon l'invention est caractérisée en ce qu'elle comprend en outre une teneur massique en acide hyaluronique comprise entre 5 et 14 mg par gramme de composition (5 mg/g ≤ teneur massique en acide hyaluronique ≤ 14 mg/g).

Dans un mode de réalisation, la composition selon l'invention est caractérisée en ce que la teneur massique en acide hyaluronique est comprise entre 7 et 12 mg par gramme de composition (7 mg/g ≤ teneur massique en acide hyaluronique ≤ 12 mg/g).

Dans un mode de réalisation, la composition selon l'invention est caractérisée en ce qu'elle comprend en outre une teneur massique en facteur de croissance TGF-β1 comprise entre 0,6 et 1,9 ng par gramme de composition (0,6 ng/g ≤ teneur massique en TGF-β1 ≤ 1,9 ng/g).

Dans un mode de réalisation, la composition selon l'invention est caractérisée en ce qu'elle comprend en outre une teneur massique en facteur de croissance TGF-β1 comprise entre 0,8 et 1,7 ng par gramme de composition (0,8 ng/g ≤ teneur massique en TGF-β1 ≤ 1,7 ng/g).

Dans un mode de réalisation, la composition selon l'invention est caractérisée en ce qu'elle comprend en outre un principe actif choisi dans le groupe constitué des antibiotiques, des antiseptiques, des antiviraux, des anticorps monoclonaux, des inhibiteurs semi-synthétiques des métalloprotéases, des agents immunosuppresseurs, des anti-inflammatoires, des anti-cancéreux, des antifongiques, des anti-allergiques, des anesthésiques, ou des protéines immunoadhésives, seuls ou en combinaisons.

La composition selon l'invention est caractérisée en ce qu'elle comprend en outre un principe actif choisi dans le groupe constitué des antibiotiques. Quelques exemples d'antibiotiques utilisables sont donnés ci-après : tétracyclines (daunomycine, tétracycline, chloretetracycline, oxytétracycline, etc.), glycopeptides (vancomycine, etc.), aminoglycosides (gentamycine, etc.), aminosides (tobramycine, néomycine, etc.), fluoroquinolones (ciprofloxacine, moxifloxacin, etc.), quinolones (gatifloxacine, etc.), polypeptides (bacitracine, polymyxine, etc.), phénicolés (chloramphénicol, etc.), macrolides (erythromycine, etc.), sulfonamides (sulfacétamide, sulfaméthoxazole, sulfisoxazole, etc.), céphalosporines (céfradoxil, céfoxitine, etc.), et tout autre antibiotique.

La composition selon l'invention est caractérisée en ce qu'elle comprend en outre un principe actif choisi dans le groupe constitué des anti-inflammatoires stéroïdiens (AIS) et/ou non-stéroïdiens (AINS). Quelques exemples d'AIS sont donnés ci-après : triamcinolone, dexaméthasone, prednisolone, hydrocortisone, corticostérone, fluocinolone, prednisolone, méthylprednisolone, fluorométholone, betaméthasone, tétrahydrocortisol, riméxolone, etc. Quelques exemples d'AINS sont donnés ci-après : indométacine, népafénac, diclofénac, bromfénac, kétorolac, suprofène, etc.

La composition selon l'invention est caractérisée en ce qu'elle comprend en outre un principe actif choisi dans le groupe constitué des anticancéreux, une liste non-choisi dans le groupe comprenant de la poudre d'os minéralisé, de la poudre d'os déminéralisé, de la poudre de calcium, de la poudre de sulfate de calcium, de la poudre de phosphate de calcium, de la poudre de corail, de la poudre d'hydroxyapatite, de la poudre de céramique, de la poudre de bioverre, seule ou en combinaisons.

Selon un mode de réalisation, la composition selon l'invention est caractérisée en ce qu'elle comprend en outre un substitut osseux sous forme de poudre qui est de la poudre d'os minéralisé.

Selon un mode de réalisation, la composition selon l'invention est caractérisée en ce qu'elle comprend en outre un substitut osseux sous forme de poudre qui est de la poudre d'os spongieux minéralisé.

Dans un mode de réalisation, la composition selon l'invention est caractérisée en ce qu'elle comprend en outre un substitut osseux sous forme de poudre qui est de la poudre d'os spongieux minéralisé d'origine humaine.

Dans un mode de réalisation, la composition selon l'invention est caractérisée en ce qu'elle comprend en outre un substitut osseux sous forme de poudre dont la granulométrie des éléments constitutifs dudit substitut osseux sous forme de poudre est comprise entre 0,1 et 2,2 mm (0,1 mm ≤ granulométrie ≤ 2,2 mm).

Dans un mode de réalisation, la composition selon l'invention est caractérisée en ce qu'elle comprend en outre un substitut osseux sous forme de poudre dont la granulométrie des éléments constitutifs dudit substitut osseux sous forme de poudre est comprise entre 0,2 et 2,0 mm (0,2 mm ≤ granulométrie ≤ 2,0 mm).

Dans la présente demande, il est entendu que les éléments constitutifs dudit substitut osseux sous forme de poudre n'ont pas tous la même granulométrie, mais une variété de dimensions incluses dans les intervalles précédemment indiqués.

Dans un mode de réalisation, la composition selon l'invention est caractérisée en ce qu'elle comprend en outre un substitut osseux sous forme de poudre en une proportion en poids de la composition comprise entre 10 % et 90 % (10 % ≤ proportion en poids du substitut osseux sous forme de poudre dans la composition ≤ 90 %).

Dans un mode de réalisation, la composition selon l'invention est caractérisée en ce qu'elle comprend en outre un substitut osseux sous forme de poudre en une proportion en poids de la composition comprise entre 20 % et 80 % (20 % ≤ proportion en poids du substitut osseux sous forme de poudre dans la composition ≤ 80 %).

Dans un mode de réalisation, la composition selon l'invention est caractérisée en ce qu'elle comprend en outre un substitut osseux sous forme de poudre en une proportion en poids de la composition comprise entre 25 % et 75 % (25 % ≤ proportion en poids du substitut osseux sous forme de poudre dans la composition ≤ 75 %).

Dans un mode de réalisation, la composition selon l'invention est caractérisée en ce qu'elle comprend en outre un substitut osseux sous forme de poudre en une proportion en poids de la composition d'environ 25 %.

Dans un mode de réalisation, la composition selon l'invention est caractérisée en ce qu'elle comprend en outre un substitut osseux sous forme de poudre en une proportion en poids de la composition d'environ 50 %.

Dans un mode de réalisation, la composition selon l'invention est caractérisée en ce qu'elle comprend en outre un substitut osseux sous forme de poudre en une proportion en poids de la composition d'environ 75 %.

Dans un mode de réalisation, la composition selon l'invention est caractérisée en ce qu'elle est moulée.

Par « moulé », on entend que la composition lyophilisée est définie par une forme qui est celle du contenant dans lequel la composition a été lyophilisée, à savoir un moule.

Dans un mode de réalisation, la composition selon l'invention est caractérisée en ce qu'elle est sous une forme comprise dans le groupe constitué du parallélépipède, du cylindre, du cône et de la sphère.

Dans un mode de réalisation, la composition selon l'invention est caractérisée en ce qu'elle est sous forme de parallélépipède dont la longueur et la largeur sont comprises entre 0,2 cm et 10,0 cm et dont la hauteur est comprise entre 0,2 cm et 1,0 cm.

Dans un mode de réalisation, la composition selon l'invention est caractérisée en ce qu'elle est sous forme de cylindre dont le diamètre est compris entre 0,2 cm et 10,0 cm et dont la hauteur est comprise entre 0,2 cm et 1,0 cm.

Dans un mode de réalisation, la composition selon l'invention est caractérisée en ce qu'elle est sous forme de sphère dont le diamètre est compris entre 0,2 cm et 1,0 cm.

Dans un mode de réalisation, la composition selon l'invention est caractérisée en ce qu'elle est sous forme de cône dont le diamètre du plan est compris entre 0,2 et 1,0 cm et dont la hauteur est comprise entre 0,2 cm et 1,0 cm.

Dans un mode de réalisation, la composition selon l'invention est caractérisée en ce qu'elle est conditionnée sous emballage stérile.

Dans un mode de réalisation, la composition selon l'invention est caractérisée en ce qu'elle est conditionnée dans un moule, ledit moule étant choisi dans le groupe comprenant un moule en verre, un moule en polyéthylène téréphtalate glycolysé (PETG), un moule en polyéthylène (PE), un moule en copolymère PE-PET, un moule en inox.

Dans un mode de réalisation, le moule en inox est un moule en inox 316L (selon la norme américaine AISI : 316L ; selon la norme européenne EN 10027 : X2CrNiMol7-12-02 1.4404).

Les moules de conditionnement peuvent être les mêmes que ceux utilisés lors de l'étape de lyophilisation de la composition lorsque cette dernière est lyophilisée.

Les moules de conditionnement peuvent être différents de ceux utilisés lors de l'étape de lyophilisation de la composition lorsque cette dernière est lyophilisée.

Le conditionnement peut être caractérisé en ce qu'il permet une dispensation rythmée de la composition par heure ou par jour.

Lorsque la composition selon l'invention est lyophilisée, celle-ci acquière un aspect mousseux à texture cohésive. La forme prise par le broyat de gelée de Wharton viro-inactivée et lyophilisée est donc celle du moule dans lequel le broyat est lyophilisé. La forme lyophilisée de l'invention permet une manipulation avantageuse par le praticien pour positionner la composition *in situ* chez le patient. Ce positionnement *in situ* au site de la lésion tissulaire, ou du comblement osseux, ou du comblement d'une alvéole dentaire, permet une diffusion locale des éléments constitutifs de la gelée de Wharton et des principes actifs pouvant être inclus dans la composition.

L'invention peut donc également être définie comme un lyophilisat moulé comprenant la composition telle que définie précédemment et qui est un broyat de gelée de Wharton viro-inactivée.

Par « moulé », on entend que le lyophilisat est défini par une forme qui est celle du contenant dans lequel la composition a été lyophilisée, à savoir un moule.

Dans un mode de réalisation, le lyophilisat moulé comprend une composition selon l'invention, caractérisé par une forme comprise dans le groupe constitué du parallélépipède, du cylindre, de la sphère et du cône.

Dans un mode de réalisation, le lyophilisat moulé comprend une composition selon l'invention, caractérisé par une forme de parallélépipède dont la longueur et la largeur sont comprises entre 0,2 cm et 10,0 cm et dont la hauteur est comprise entre 0,2 cm et 1,0 cm.

Dans un mode de réalisation, le lyophilisat moulé comprend une composition selon l'invention, caractérisé par une forme de cylindre dont le diamètre est compris entre 0,2 cm et 10,0 cm et dont la hauteur est comprise entre 0,2 cm et 1,0 cm.

Dans un mode de réalisation, le lyophilisat moulé comprend une composition selon l'invention, caractérisé par une forme de sphère dont le diamètre est compris entre 0,2 cm et 1,0 cm.

Dans un mode de réalisation, le lyophilisat moulé comprend une composition selon l'invention, caractérisé par une forme de cône dont le diamètre du plan est compris entre 0,2 et 1,0 cm et dont la hauteur est comprise entre 0,2 et 1,0 cm.

Dans un mode de réalisation, le lyophilisat moulé comprend une composition selon l'invention, caractérisé en ce qu'il est conditionné sous emballage stérile.

Dans un mode de réalisation, le lyophilisat moulé comprend une composition selon l'invention, caractérisé en ce qu'il est conditionné dans un moule, ledit moule étant choisi dans le groupe comprenant un moule en verre, un moule en polyéthylène téréphtalate glycolysé (PETG), un moule en polyéthylène glycol (PE), un moule en copolymère PE-PET, un moule en inox.

Dans un mode de réalisation, le moule en inox est un moule en inox 316L (selon la norme américaine AISI : 316L ; selon la norme européenne EN 10027 : X2CrNiMo17-12-02 1.4404).

Dans un mode de réalisation, la composition selon l'invention est caractérisée en ce qu'elle est stérilisée.

Dans un mode de réalisation, la composition selon l'invention est caractérisée en ce qu'elle est stérilisée par irradiation gamma.

Dans un mode de réalisation, la composition selon l'invention est caractérisée en ce qu'elle est destinée à être utilisée dans le traitement des lésions tissulaires.

Dans un mode de réalisation, la composition selon l'invention est caractérisée en ce qu'elle est destinée à être utilisée dans le traitement des lésions de la surface oculaire, le traitement d'une lésion cutanée.

Dans un mode de réalisation, l'invention concerne une composition pour le traitement des lésions de la surface oculaire.

Dans un mode de réalisation, l'invention concerne une composition pour le traitement d'une lésion cutanée.

Les lésions cutanées peuvent être définies de manière non limitative par les lésions cutanées aigues ou chroniques allergiques, les infections cutanées, les ulcères variqueux ou diabétiques, les brûlures cutanées, les plaies cutanées, les plaies de cicatrisation cutanées.

Les lésions oculaires peuvent être définies de manière non limitative par les inflammations ou infections de la surface oculaire, le syndrome de l'œil sec, l'ulcère.

Dans un mode de réalisation, la composition selon l'invention est caractérisée en ce qu'elle est destinée à être utilisée dans le comblement osseux.

Dans un mode de réalisation, l'invention concerne une composition pour le traitement des pertes osseuses, pour le traitement de l'arthrose, pour permettre une fusion osseuse dans le cadre d'une arthrodèse.

Les pertes osseuses peuvent être définies de manière non limitative par des pertes de tissu osseux par rapport à un tissu osseux sain. A titre d'exemple, une perte osseuse peut survenir suite à une trépanation crânienne.

Dans ce mode de réalisation, l'utilisation de la composition selon l'invention dans le comblement osseux est particulièrement appréciée. En effet, la souplesse de la composition selon l'invention permet au praticien de placer aisément celle-ci au site nécessitant un comblement osseux chez un patient. Également, la composition conserve l'enchâssement de l'os, même après réhydratation de la composition lorsque celle-ci est utilisée lyophilisée. La composition selon l'invention, destinée à être utilisée dans le comblement osseux, offre donc une matrice de support stable dans laquelle la cicatrisation osseuse va pouvoir s'effectuer de façon propice.

Dans un mode de réalisation, la composition selon l'invention est caractérisée en ce qu'elle est destinée à être utilisée dans le comblement des alvéoles dentaires.

Dans un mode de réalisation, l'invention concerne une composition pour le comblement des alvéoles dentaires.

Les alvéoles dentaires peuvent être définies de manière non limitative par les cavités présentes dans le maxillaire (supérieur ou inférieur) dans lesquelles viennent se loger les dents.

Par comblement des alvéoles dentaires, on entend de manière non limitative l'acte de combler les cavités des alvéoles dentaires laissées vides suite à la perte des dents associées auxdites alvéoles dentaires. Cette perte des dents pouvant par exemple faire suite à un traumatisme ou un acte chirurgical.

Dans ce mode de réalisation, l'utilisation de la composition selon l'invention est particulièrement appréciée en ce que la composition permet la conservation de la forme des alvéoles dentaires tout en améliorant la cicatrisation du tissu.

Ainsi, la régénération de l'os maxillaire est guidée et améliorée par la composition utilisée comme solution de comblement. Cette régénération guidée et améliorée permet l'obtention d'un tissu stable permettant notamment la pose d'un implant dentaire.

Dans un mode de réalisation, la composition selon l'invention est caractérisée en ce qu'elle est destinée à être utilisée dans l'amélioration de la cicatrisation, la réduction de l'inflammation, l'amélioration de l'hydratation par des propriétés hygroscopiques.

Dans un mode de réalisation, la composition selon l'invention est caractérisée en ce qu'elle est destinée à être utilisée sans réhydratation préalable à son utilisation.

Lorsque la composition est utilisée sans réhydratation préalable, la réhydratation peut s'effectuer in situ par hydratation naturelle par un fluide issu du corps du patient.

Dans un mode de réalisation, la composition selon l'invention est caractérisée en ce qu'elle est destinée à être utilisée après réhydratation préalable à son utilisation.

Le procédé de production du biomatériau consistant en un broyat homogène de gelée de Wharton viro-inactivée est ci-après décrit. Le procédé de production dudit biomatériau peut également comprendre une étape de lyophilisation permettant l'obtention d'un biomatériau consistant en un broyat homogène de gelée de Wharton viro-inactivée, caractérisé en ce qu'il est lyophilisé.

Le procédé de production concerne également une composition comprenant au moins ledit biomatériau.

Le procédé de production de ladite composition comprenant au moins ledit biomatériau peut également comprendre une étape de lyophilisation permettant l'obtention d'une composition comprenant au moins ledit biomatériau, caractérisée en ce qu'elle est lyophilisée.

L'invention concerne donc également un procédé de préparation d'une composition, ou d'un lyophilisat moulé tels que définis précédemment, caractérisé en ce qu'il comprend au moins les étapes suivantes :
a) on dispose d'un segment de gelée de Wharton dont la veine et les artères ont été ôtées ;
b) on effectue un traitement de viro-inactivation du segment de gelée de Wharton pour obtenir un segment de gelée de Wharton viro-inactivée ;
c) on effectue au moins une étape de broyage du segment de gelée de Wharton viro-inactivée pour obtenir un broyat homogène de gelée de Wharton viro-inactivée.

Dans un mode de réalisation, le procédé selon l'invention est caractérisé en ce qu'il comporte en outre une étape d), postérieure à l'étape c), définie comme suit :
d) on effectue une lyophilisation du broyat de gelée de Wharton viro-inactivée pour obtenir un broyat de gelée de Wharton viro-inactivée et lyophilisée.

Dans un mode de réalisation, le procédé selon l'invention est caractérisé en ce qu'il comporte en outre une étape e), postérieure à l'étape c), définie comme suit :
e) on effectue au moins une étape de tamisage du broyat de gelée de Wharton viro-inactivée et lyophilisée.

Dans un mode de réalisation, le procédé selon l'invention est caractérisé en ce que les étapes a), b) et c) s'effectuent à température ambiante.

Dans un mode de réalisation, le procédé selon l'invention est caractérisé en ce que le traitement de viro-inactivation comprend au moins deux étapes successives ((x) et (y)) de traitement par un agent de viro-inactivation chimique.

Dans un mode de réalisation, le procédé selon l'invention est caractérisé en ce que l'agent de viro-inactivation chimique est choisi dans le groupe constitué par les alcools et/ou les peroxydes.

En effet, la demanderesse a démontré l'efficacité d'une viro-inactivation chimique en deux étapes : une étape de traitement chimique avec un alcool particulièrement efficace envers les virus à enveloppes ; une étape de traitement chimique avec un peroxyde particulièrement efficace envers les virus nus (voir Exemples 2 et 3).

Ainsi, la combinaison successive des deux étapes permet d'obtenir une viro-inactivation du tissu biologique, de telle sorte à diminuer la population virale de 4 log lors des titrages résiduels, envers tous les virus, qu'ils soient enveloppés ou nus.

Dans un mode de réalisation, le procédé selon l'invention est caractérisé en ce que la première étape de traitement (x) est effectuée par un agent de viro-inactivation choisi dans le groupe constitué par les alcools.

Dans un mode de réalisation, le procédé selon l'invention est caractérisé en ce que le premier agent de viro-inactivation est l'éthanol à une teneur en alcool comprise entre 50 % et 80 % v/v.

Dans un mode de réalisation, le procédé selon l'invention est caractérisé en ce que le premier agent de viro-inactivation est l'éthanol à 70 % v/v.

Dans un mode de réalisation, le procédé selon l'invention est caractérisé en ce que la première étape de traitement (x) est effectuée pendant une durée comprise entre 40 minutes et 120 minutes.

Dans un mode de réalisation, le procédé selon l'invention est caractérisé en ce que la première étape de traitement (x) est effectuée pendant une durée de 60 minutes.

Dans un mode de réalisation, le procédé selon l'invention est caractérisé en ce que la deuxième étape de traitement (y) est effectuée par un agent de viro-inactivation choisi dans le groupe constitué par les peroxydes.

Dans un mode de réalisation, le procédé selon l'invention est caractérisé en ce que le deuxième agent de viro-inactivation est le peroxyde d'hydrogène.

Dans un mode de réalisation, le procédé selon l'invention est caractérisé en ce que le deuxième agent de viro-inactivation est le peroxyde d'hydrogène sous une forme choisie parmi une solution aqueuse et un gaz.

Dans un mode de réalisation, le procédé selon l'invention est caractérisé en ce que le deuxième agent de viro-inactivation est le peroxyde d'hydrogène sous forme de solution aqueuse dans une concentration comprise entre 1 % et 40 % w/v.

Dans un mode de réalisation, le procédé selon l'invention est caractérisé en ce que la deuxième étape de viro-inactivation (y) comporte deux sous-étapes de traitement ((q) et (r)) par le peroxyde d'hydrogène :
- une première étape de traitement (q) s'effectuant avec une solution de peroxyde d'hydrogène à une concentration comprise entre 20 % et 40 % w/v pendant 10 à 20 minutes ;
- une deuxième étape de traitement (r) s'effectuant avec une solution de peroxyde d'hydrogène à une concentration comprise entre 1 % et 10 % w/v pendant 40 à 80 minutes.

Dans un mode de réalisation, le procédé selon l'invention est caractérisé en ce que le deuxième agent de viro-inactivation est le peroxyde d'hydrogène est sous forme de solution aqueuse dans une concentration comprise entre 3 % et 30 % w/v.

Dans un mode de réalisation, le procédé selon l'invention est caractérisé en ce que la deuxième étape de viro-inactivation (y) comporte deux sous-étapes de traitement, (q) et (r), par le peroxyde d'hydrogène :
- une première étape de traitement (q) s'effectuant avec une solution de peroxyde d'hydrogène à 30 % w/v pendant 10 à 20 minutes ;
- une deuxième étape de traitement (r) s'effectuant avec une solution de peroxyde d'hydrogène à 3 % w/v pendant 40 à 80 minutes.

Dans un mode de réalisation, le procédé selon l'invention est caractérisé en ce que la deuxième étape de viro-inactivation (y) comporte deux sous-étapes de traitement, (q) et (r), par le peroxyde d'hydrogène :
- une première étape de traitement (q) s'effectuant avec une solution de peroxyde d'hydrogène à 30 % w/v pendant 15 minutes ;
- une deuxième étape de traitement (r) s'effectuant avec une solution de peroxyde d'hydrogène à 3 % w/v pendant 60 minutes.

Dans un mode de réalisation, le procédé selon l'invention est caractérisé en ce qu'il comporte en outre au moins une étape lors de laquelle le segment de gelée de Wharton est lavé à l'eau purifiée entre les étapes (x) et (y) de viro-inactivation chimique.

Dans un mode de réalisation, le procédé selon l'invention est caractérisé en ce qu'il comporte en outre au moins une étape d'ajustement du pH de la solution dans laquelle se trouve le segment de gelée de Wharton viro-inactivée entre les étapes b) et c).

Dans un mode de réalisation, le procédé selon l'invention est caractérisé en ce qu'il comporte en outre au moins une étape d'ajustement du pH entre 7 et 9 de la solution dans laquelle se trouve le segment de gelée de Wharton viro-inactivée entre les étapes b) et c).

Dans un mode de réalisation, le procédé selon l'invention est caractérisé en ce qu'il comporte en outre au moins une étape d'ajustement du pH à 8,5 de la solution dans laquelle se trouve le segment de gelée de Wharton viro-inactivée entre les étapes b) et c).

Dans un mode de réalisation, le procédé selon l'invention est caractérisé en ce qu'il comporte en outre au moins une étape d'ajustement du pH de la solution dans laquelle se trouve le segment de gelée de Wharton viro-inactivée, suivie d'au moins une étape de mise en tampon du segment de gelée de Wharton viro-inactivée entre les étapes b) et c).

Dans un mode de réalisation, le procédé selon l'invention est caractérisé en ce que l'étape de mise en tampon est définie par deux bains successifs de solution tampon de PBS (tampon phosphate salin).

Dans un mode de réalisation, le procédé selon l'invention est caractérisé en ce que l'étape de mise en tampon est suivie de deux étapes de lavage en bains d'eau purifiée.

Dans un mode de réalisation, le procédé selon l'invention est caractérisé en ce que l'étape c) de broyage est effectuée par l'utilisation d'un broyeur centrifuge.

Dans un mode de réalisation, le procédé selon l'invention est caractérisé en ce que l'étape c) de broyage est effectuée par l'utilisation d'un broyeur centrifuge, sans qu'une quelconque solution ne soit ajoutée au segment de gelée de Wharton viro-inactivée lors de cette étape c) de broyage.

Cette étape c) de broyage est réalisée en ce que ledit segment de gelée de Wharton est broyé après lavage pour obtenir un gel.

Par l'utilisation de l'expression « sans qu'une quelconque solution ne soit ajoutée », il est convenu que seule la fraction liquide naturellement présente dans le tissu biologique, à savoir la fraction liquide imbibée dans ledit tissu après les deux étapes de lavage en bains d'eau purifiée est présente lors de l'étape c) de broyage. Cette fraction liquide imbibée représente moins de 5% en poids du segment de gelée de Wharton après les deux étapes de lavage en bains d'eau purifiée.

La demanderesse a démontré que l'utilisation d'un broyeur centrifuge lors d'une des étapes de broyage permettait d'obtenir un broyat homogène (voir Exemple 4). Cette caractéristique d'homogénéité du broyat permet ainsi une préservation des composants du tissu biologique initial, qui est la gelée de Wharton, dans le produit fini. Ainsi, il n'est pas nécessaire d'effectuer d'étape de filtration pour éliminer une partie de la composition qui n'aurait pas été réduite lors du broyage.

Dans un mode de réalisation, le procédé selon l'invention est caractérisé en ce que l'étape c) de broyage est effectuée par l'utilisation d'un broyeur centrifuge dont la maille du tamis est inférieure à 3 mm.

Dans un mode de réalisation, le procédé selon l'invention est caractérisé en ce que l'étape c) de broyage est effectuée par l'utilisation d'un broyeur centrifuge dont la maille du tamis est inférieure à 0,5 mm.

Dans un mode de réalisation, le procédé selon l'invention est caractérisé en ce que l'étape c) de broyage est effectuée par l'utilisation d'un broyeur centrifuge dont la maille du tamis est comprise entre 0,03 et 3 mm.

Dans un mode de réalisation, le procédé selon l'invention est caractérisé en ce que l'étape c) de broyage est effectuée par l'utilisation d'un broyeur centrifuge dont la maille du tamis est comprise entre 0,05 et 3 mm.

Dans un mode de réalisation, le procédé selon l'invention est caractérisé en ce que l'étape c) de broyage est effectuée par l'utilisation d'un broyeur centrifuge dont la maille du tamis est comprise entre 0,08 et 3 mm.

Dans un mode de réalisation, le procédé selon l'invention est caractérisé en ce que l'étape c) de broyage est effectuée par l'utilisation d'un broyeur centrifuge dont la maille du tamis est de 3 mm.

Dans un mode de réalisation, le procédé selon l'invention est caractérisé en ce que l'étape c) de broyage est effectuée par l'utilisation d'un broyeur centrifuge dont la maille du tamis est de 3 mm puis 1 mm.

La demanderesse a démontré que des étapes de broyage effectuées par l'utilisation d'un broyeur centrifuge équipé de tamis dont les mailles sont de tailles décroissantes permettent un rendement amélioré lorsque l'obtention d'un broyat constitué de particules fines est souhaitée (voir Exemple 5).

Dans un mode de réalisation, le procédé selon l'invention est caractérisé en ce que l'étape c) de broyage est effectuée par l'utilisation d'un broyeur centrifuge dont la maille du tamis est de 1 mm.

Dans un mode de réalisation, le procédé selon l'invention est caractérisé en ce que l'étape c) de broyage est effectuée par l'utilisation d'un broyeur centrifuge dont la maille du tamis est inférieure à 1 mm.

Dans un mode de réalisation, le procédé selon l'invention est caractérisé en ce que l'étape c) de broyage est effectuée par l'utilisation d'un broyeur centrifuge dont la maille du tamis est de 0,08 mm.

Lorsque la composition obtenue selon le procédé est destinée à être utilisée dans la bio-impression, un tamis dont la maille est inférieure ou égale à 1 mm est utilisé.

Dans un mode de réalisation, le procédé selon l'invention est caractérisé en ce que l'étape c) de broyage est effectuée par l'utilisation d'un broyeur centrifuge à une vitesse de centrifugation comprise entre 6 000 tours/minute et 20 000 tours/minute pendant au moins 30 secondes.

Dans un mode de réalisation, le procédé selon l'invention est caractérisé en ce que l'étape c) de broyage est effectuée par l'utilisation d'un broyeur centrifuge à une vitesse de centrifugation comprise entre 6 000 tours/minute et 20 000 tours/minute pendant une durée comprise entre 30 secondes et 3 minutes.

Dans un mode de réalisation, le procédé selon l'invention est caractérisé en ce que l'étape c) de broyage est effectuée par l'utilisation d'un broyeur centrifuge à une vitesse de centrifugation de 10 000 tours/minute pendant 1 minute.

Dans un mode de réalisation, le procédé selon l'invention est caractérisé en ce que l'étape c) de broyage est effectuée par l'utilisation d'un broyeur vibrant à billes.

Dans un mode de réalisation, le procédé selon l'invention est caractérisé en ce que l'étape c) de broyage est effectuée par l'utilisation d'un broyeur vibrant à billes, sans qu'une quelconque solution ne soit ajoutée au segment de gelée de Wharton viro-inactivée lors de cette étape c) de broyage.

La demanderesse a démontré que l'utilisation d'un broyeur vibrant à billes lors d'une des étapes de broyage permettait d'obtenir un broyat homogène. Cette caractéristique d'homogénéité du broyat permet ainsi une préservation des composants du tissu biologique initial, qui est la gelée de Wharton, dans le produit fini. Ainsi, il n'est pas nécessaire d'effectuer d'étape de filtration pour éliminer une partie de la composition qui n'aurait pas été réduite lors du broyage.

Dans un mode de réalisation, le procédé selon l'invention est caractérisé en ce que l'étape c) de broyage est effectuée par l'utilisation d'un broyeur vibrant à billes à une fréquence de vibration d'au moins 1 Hz.

Dans un mode de réalisation, le procédé selon l'invention est caractérisé en ce que l'étape c) de broyage est effectuée par l'utilisation d'un broyeur vibrant à billes à une fréquence de vibration d'au moins 3 Hz.

Dans un mode de réalisation, le procédé selon l'invention est caractérisé en ce que l'étape c) de broyage est effectuée par l'utilisation d'un broyeur vibrant à billes à une fréquence de vibration comprise entre 1 et 50 Hz.

Dans un mode de réalisation, le procédé selon l'invention est caractérisé en ce que l'étape c) de broyage est effectuée par l'utilisation d'un broyeur vibrant à billes à une fréquence de vibration comprise entre 2 et 40 Hz.

Dans un mode de réalisation, le procédé selon l'invention est caractérisé en ce que l'étape c) de broyage est effectuée par l'utilisation d'un broyeur vibrant à billes à une fréquence de vibration comprise entre 3 et 30 Hz.

Dans un mode de réalisation, le procédé selon l'invention est caractérisé en ce que l'étape c) de broyage est effectuée par l'utilisation d'un broyeur vibrant à billes à une fréquence de vibration de 3 Hz.

Dans un mode de réalisation, le procédé selon l'invention est caractérisé en ce que l'étape c) de broyage est effectuée par l'utilisation d'un broyeur vibrant à billes à une fréquence de vibration de 30 Hz.

Dans un mode de réalisation, le procédé selon l'invention est caractérisé en ce que l'étape c) de broyage est effectuée par l'utilisation d'un broyeur vibrant à billes pendant une durée de broyage d'au moins 30 secondes.

Dans un mode de réalisation, le procédé selon l'invention est caractérisé en ce que l'étape c) de broyage est effectuée par l'utilisation d'un broyeur vibrant à billes pendant une durée de broyage comprise entre 30 secondes et 5 minutes.

Dans un mode de réalisation, le procédé selon l'invention est caractérisé en ce que l'étape c) de broyage est effectuée par l'utilisation d'un broyeur vibrant à billes pendant une durée de broyage comprise entre 1 minute et 3 minutes.

Dans un mode de réalisation, le procédé selon l'invention est caractérisé en ce que l'étape c) de broyage est effectuée par l'utilisation d'un broyeur vibrant à billes pendant une durée de broyage de 1 minute.

Dans un mode de réalisation, le procédé selon l'invention est caractérisé en ce que l'étape c) de broyage est effectuée par l'utilisation d'un broyeur vibrant à billes pendant une durée de broyage de 3 minutes.

Dans un mode de réalisation, le procédé selon l'invention est caractérisé en ce que l'étape c) de broyage est effectuée par l'utilisation d'un broyeur vibrant à billes, dont les billes sont en oxyde de zirconium.

Dans un mode de réalisation, le procédé selon l'invention est caractérisé en ce que l'étape c) de broyage est effectuée par l'utilisation d'un broyeur vibrant à billes, dont les billes et le bol de broyage sont en oxyde de zirconium.

Dans un mode de réalisation, le procédé selon l'invention est caractérisé en ce que l'étape c) de broyage est effectuée par l'utilisation d'un broyeur vibrant à billes, dont les billes ont un diamètre d'au moins 5 mm.

Dans un mode de réalisation, le procédé selon l'invention est caractérisé en ce que l'étape c) de broyage est effectuée par l'utilisation d'un broyeur vibrant à billes, dont les billes ont un diamètre compris entre 5 et 30 mm.

Dans un mode de réalisation, le procédé selon l'invention est caractérisé en ce que l'étape c) de broyage est effectuée par l'utilisation d'un broyeur vibrant à billes, dont les billes ont un diamètre compris entre 10 et 20 mm.

Dans un mode de réalisation, le procédé selon l'invention est caractérisé en ce que l'étape c) de broyage est effectuée par l'utilisation d'un broyeur vibrant à billes, dont les billes ont un diamètre de 10 mm.

Dans un mode de réalisation, le procédé selon l'invention est caractérisé en ce que l'étape c) de broyage est effectuée par l'utilisation d'un broyeur vibrant à billes, dont les billes ont un diamètre de 20 mm.

Dans un mode de réalisation, le procédé selon l'invention est caractérisé en ce que l'étape c) de broyage est effectuée par l'utilisation d'un broyeur vibrant à billes, avec 1 à 20 billes.

Dans un mode de réalisation, le procédé selon l'invention est caractérisé en ce que l'étape c) de broyage est effectuée par l'utilisation d'un broyeur vibrant à billes, avec une bille dont le diamètre est compris entre 5 et 30 mm.

Dans un mode de réalisation, le procédé selon l'invention est caractérisé en ce que l'étape c) de broyage est effectuée par l'utilisation d'un broyeur vibrant à billes, avec une bille dont le diamètre est compris entre 15 et 25 mm.

Dans un mode de réalisation, le procédé selon l'invention est caractérisé en ce que l'étape c) de broyage est effectuée par l'utilisation d'un broyeur vibrant à billes, avec une bille dont le diamètre est de 20 mm.

Dans un mode de réalisation, le procédé selon l'invention est caractérisé en ce que l'étape c) de broyage est effectuée par l'utilisation d'un broyeur vibrant à billes, avec entre 5 et 15 billes dont le diamètre est compris entre 5 et 15 mm.

Dans un mode de réalisation, le procédé selon l'invention est caractérisé en ce que l'étape c) de broyage est effectuée par l'utilisation d'un broyeur vibrant à billes, avec entre 5 et 15 billes dont le diamètre est compris entre 7 et 12 mm.

Dans un mode de réalisation, le procédé selon l'invention est caractérisé en ce que l'étape c) de broyage est effectuée par l'utilisation d'un broyeur vibrant à billes, avec entre 5 et 15 billes dont le diamètre est de 10 mm.

Dans un mode de réalisation, le procédé selon l'invention est caractérisé en ce que l'étape c) de broyage est effectuée par l'utilisation d'un broyeur vibrant à billes, avec entre 8 et 12 billes dont le diamètre est de 10 mm.

Dans un mode de réalisation, le procédé selon l'invention est caractérisé en ce que l'étape c) de broyage est effectuée par l'utilisation d'un broyeur vibrant à billes, avec 9 billes dont le diamètre est de 10 mm.

Dans un mode de réalisation, le procédé selon l'invention est caractérisé en ce que l'étape c) de broyage est effectuée par l'utilisation d'un broyeur vibrant à billes, à une fréquence de vibration comprise entre 1 et 50 Hz, pendant une durée de broyage comprise entre 30 secondes et 5 minutes, avec 1 à 20 billes, dont les billes ont un diamètre compris entre 5 et 30 mm.

Dans un mode de réalisation, le procédé selon l'invention est caractérisé en ce que l'étape c) de broyage est effectuée par l'utilisation d'un broyeur vibrant à billes, à une fréquence de vibration comprise entre 1 et 5 Hz, pendant une durée de broyage comprise entre 30 secondes et 3 minutes, avec 1 à 5 billes, dont les billes ont un diamètre compris entre 5 et 20 mm.

Dans un mode de réalisation, le procédé selon l'invention est caractérisé en ce que l'étape c) de broyage est effectuée par l'utilisation d'un broyeur vibrant à billes, à une fréquence de vibration de 3 Hz, pendant une durée de broyage de 1 minute, avec une bille, dont la bille a un diamètre de 20 mm.

Dans un mode de réalisation, le procédé selon l'invention est caractérisé en ce que l'étape c) de broyage est effectuée par l'utilisation d'un broyeur vibrant à billes, à une fréquence de vibration comprise entre 10 et 40 Hz, pendant une durée de broyage comprise entre 30 secondes et 3 minutes, avec 5 à 12 billes, dont les billes ont un diamètre compris entre 7 et 12 mm.

Dans un mode de réalisation, le procédé selon l'invention est caractérisé en ce que l'étape c) de broyage est effectuée par l'utilisation d'un broyeur vibrant à billes, à une fréquence de vibration de 30 Hz, pendant une durée de broyage de 1 minute, avec 9 billes, dont les billes ont un diamètre de 10 mm.

Dans un mode de réalisation, le procédé selon l'invention est caractérisé en ce que l'étape c) de broyage est effectuée par l'utilisation d'un broyeur vibrant à billes, en ce que cette étape c) est définie par les sous-étapes successives de broyage suivantes :
- Une première sous-étape de broyage où le broyeur vibrant à billes est utilisé à une fréquence de vibration comprise entre 1 et 5 Hz, pendant une durée de broyage comprise entre 30 secondes et 3 minutes, avec 1 à 5 billes, dont les billes ont un diamètre compris entre 5 et 20 mm.
- Les 1 à 5 billes dont le diamètre est compris entre 5 et 20 mm sont retirées du bol de broyage du broyeur vibrant à billes. De 5 à 12 billes dont le diamètre est compris entre 7 et 12 mm sont ajoutées au bol de broyage du broyeur vibrant à billes.
- Une deuxième sous-étape de broyage, successive à la première, où le broyeur vibrant à billes est utilisé à une fréquence de vibration comprise entre 10 et 40 Hz, pendant une durée de broyage comprise entre 30 secondes et 3 minutes, avec 5 à 12 billes, dont les billes ont un diamètre compris entre 7 et 12 mm.

Dans un mode de réalisation, le procédé selon l'invention est caractérisé en ce que l'étape c) de broyage est effectuée par l'utilisation d'un broyeur vibrant à billes, en ce que cette étape c) est définie par les sous-étapes successives de broyage suivantes :
- Une première sous-étape de broyage où le broyeur vibrant à billes est utilisé à une fréquence de vibration de 3 Hz, pendant une durée de broyage de 1 minute, avec 1 bille, dont la bille a un diamètre de 20 mm.
- La 1 bille dont le diamètre est de 20 mm est retirée du bol de broyage du broyeur vibrant à billes. 9 billes dont le diamètre est de 10 mm sont ajoutées au bol de broyage du broyeur vibrant à billes.
- Une deuxième sous-étape de broyage, où le broyeur vibrant à billes est utilisé à une fréquence de vibration de 30 Hz, pendant une durée de broyage de 1 minute, avec 9 billes, dont les billes ont un diamètre de 10 mm.

Dans un mode de réalisation, le procédé selon l'invention est caractérisé en ce qu'il comporte une étape de mise en forme du broyat de gelée de Wharton viro-inactivée entre les étapes c) et d).

Dans un mode de réalisation, le procédé selon l'invention est caractérisé en ce que l'étape de mise en forme est effectuée par moulage.

Dans un mode de réalisation, le procédé selon l'invention est caractérisé en ce que le broyat de gelée de Wharton viro-inactivée est mis en forme de parallélépipède, de cylindre, de cône ou de sphère entre les étapes c) et d).

Dans un mode de réalisation, le procédé selon l'invention est caractérisé en ce que le broyat de gelée de Wharton viro-inactivée est mis en forme de parallélépipède dont la longueur et la largeur sont comprises entre 0,2 cm et 10 cm et dont la hauteur est comprise entre 0,2 cm et 1,0 cm.

Dans un mode de réalisation, le procédé selon l'invention est caractérisé en ce que le broyat de gelée de Wharton viro-inactivée est mis en forme de cylindre dont la longueur du diamètre est comprise entre 0,2 cm et 10,0 cm et dont la hauteur est comprise entre 0,2 cm et 1,0 cm.

Dans un mode de réalisation, le procédé selon l'invention est caractérisé en ce que le broyat de gelée de Wharton viro-inactivée est mis en forme de sphère dont le diamètre est compris entre 0,2 cm et 1,0 cm.

Dans un mode de réalisation, le procédé selon l'invention est caractérisé en ce que le broyat de gelée de Wharton viro-inactivée est mis en forme de cône dont le diamètre du plan est compris entre 0,2 cm et 1,0 cm et dont la hauteur est comprise entre 0,2 cm et 1,0 cm.

Dans un mode de réalisation, le procédé selon l'invention est caractérisé en ce qu'il comporte en outre une étape d'ajout d'un principe actif entre les étapes b) et c).

Dans un mode de réalisation, le procédé selon l'invention est caractérisé en ce qu'il comporte en outre une étape d'ajout d'un principe actif entre les étapes c) et d).

Dans un mode de réalisation, le procédé selon l'invention est caractérisé en ce que le principe actif est choisi dans le groupe constitué des antibiotiques, des antiseptiques, des antiviraux, des anticorps monoclonaux, des inhibiteurs semi-synthétiques des métalloprotéases, des agents immunosuppresseurs, des anti-cancéreux, des antifongiques, des anti-allergiques, des anesthésiques, ou des protéines immunoadhésives, seuls ou en combinaisons.

Quelques exemples non limitatifs de ces principes actifs sont précédemment donnés.

Dans un mode de réalisation, le procédé selon l'invention est caractérisé en ce que le principe actif est le méthotrexate (acide 4-amino-10-méthylfolique).

Dans un mode de réalisation, le procédé selon l'invention est caractérisé en ce qu'il comporte en outre une étape d'ajout d'un substitut osseux sous forme de poudre entre les étapes c) et d).

Dans un mode de réalisation, le procédé selon l'invention est caractérisé en ce qu'il comporte en outre une étape d'ajout d'un substitut osseux sous forme de poudre entre les étapes c) et d), ledit substitut osseux sous forme de poudre étant choisi dans le groupe comprenant de la poudre d'os minéralisé, de la poudre d'os déminéralisé, de la poudre de calcium, de la poudre de sulfate de calcium, de la poudre de phosphate de calcium, de la poudre de corail, de la poudre d'hydroxyapatite, de la poudre de céramique, de la poudre de bioverre, seule ou en combinaisons.

Dans un mode de réalisation, le procédé selon l'invention est caractérisé en ce qu'il comporte en outre une étape d'ajout d'un substitut osseux sous forme de poudre entre les étapes c) et d), ledit substitut osseux sous forme de poudre étant de la poudre d'os minéralisé.

Dans un mode de réalisation, le procédé selon l'invention est caractérisé en ce qu'il comporte en outre une étape d'ajout d'un substitut osseux sous forme de poudre entre les étapes c) et d), ledit substitut osseux sous forme de poudre étant de la poudre d'os spongieux minéralisé.

Dans un mode de réalisation, le procédé selon l'invention est caractérisé en ce qu'il comporte en outre une étape d'ajout d'un substitut osseux sous forme de poudre entre les étapes c) et d), ledit substitut osseux sous forme de poudre étant de la poudre d'os spongieux minéralisé d'origine humaine.

Dans un mode de réalisation, le procédé selon l'invention est caractérisé en ce qu'il comporte en outre une étape d'ajout d'un substitut osseux sous forme de poudre entre les étapes c) et d), ledit substitut osseux sous forme de poudre est mélangé de façon homogène avec le broyat homogène de gelée de Wharton viro-inactivée.

Dans un mode de réalisation, le procédé selon l'invention est caractérisé en ce qu'il comporte en outre une étape d'ajout d'un substitut osseux sous forme de poudre entre les étapes c) et d), dont la granulométrie des éléments constitutifs dudit substitut osseux sous forme de poudre est comprise entre 0,1 et 2,2 mm (0,1 mm ≤ granulométrie ≤ 2,2 mm).

Dans un mode de réalisation, le procédé selon l'invention est caractérisé en ce qu'il comporte en outre une étape d'ajout d'un substitut osseux sous forme de poudre entre les étapes c) et d), dont la granulométrie des éléments constitutifs dudit substitut osseux sous forme de poudre est comprise entre 0,2 et 2,0 mm (0,2 mm ≤ granulométrie ≤ 2,0 mm).

Dans un mode de réalisation, le procédé selon l'invention est caractérisé en ce qu'il comporte en outre une étape d'ajout d'un substitut osseux sous forme de poudre entre les étapes c) et d), dont le ratio en poids dudit substitut osseux sous forme de poudre par rapport au poids du broyat homogène de gelée de Wharton viro-inactivée est compris entre 1:9 et 9:1 (1:9 ≤ ratio en poids dudit substitut osseux sous forme de poudre par rapport au poids du broyat homogène de gelée de Wharton viro-inactivée ≤ 9:1).

Dans un mode de réalisation, le procédé selon l'invention est caractérisé en ce qu'il comporte en outre une étape d'ajout d'un substitut osseux sous forme de poudre entre les étapes c) et d), dont le ratio en poids dudit substitut osseux sous forme de poudre par rapport au poids du broyat homogène de gelée de Wharton viro-inactivée est compris entre 1:4 et 4:1 (1:4 ≤ ratio en poids dudit substitut osseux sous forme de poudre par rapport au poids du broyat homogène de gelée de Wharton viro-inactivée ≤ 4:1).

Dans un mode de réalisation, le procédé selon l'invention est caractérisé en ce qu'il comporte en outre une étape d'ajout d'un substitut osseux sous forme de poudre entre les étapes c) et d), dont le ratio en poids dudit substitut osseux sous forme de poudre par rapport au poids du broyat homogène de gelée de Wharton viro-inactivée est compris entre 2,5:7,5 et 7,5:2,5 (2,5:7,5 ≤ ratio en poids dudit substitut osseux sous forme de poudre par rapport au poids du broyat homogène de gelée de Wharton viro-inactivée ≤ 7,5:2,5).

Dans un mode de réalisation, le procédé selon l'invention est caractérisé en ce qu'il comporte en outre une étape d'ajout d'un substitut osseux sous forme de poudre entre les étapes c) et d), dont le ratio en poids dudit substitut osseux sous forme de poudre par rapport au poids du broyat homogène de gelée de Wharton viro-inactivée est d'environ 2,5:7,5.

Dans un mode de réalisation, le procédé selon l'invention est caractérisé en ce qu'il comporte en outre une étape d'ajout d'un substitut osseux sous forme de poudre entre les étapes c) et d), dont le ratio en poids dudit substitut osseux sous forme de poudre par rapport au poids du broyat homogène de gelée de Wharton viro-inactivée est d'environ 1:1.

Dans un mode de réalisation, le procédé selon l'invention est caractérisé en ce qu'il comporte en outre une étape d'ajout d'un substitut osseux sous forme de poudre entre les étapes c) et d), dont le ratio en poids dudit substitut osseux sous forme de poudre par rapport au poids du broyat homogène de gelée de Wharton viro-inactivée est d'environ 7,5:2,5.

Dans un mode de réalisation, le procédé selon l'invention est caractérisé en ce que l'étape d) de lyophilisation est effectuée selon les étapes (m) et (n) suivantes :
(m) une congélation en deux étapes (m') et (m") :
   - la première étape de congélation, (m'), s'effectuant à une température d'acclimatation choisie pour ne pas endommager l'intégralité structurelle, fonctionnelle et biologique du broyat de gelée de Wharton viro-inactivée ;
   - la deuxième étape de congélation, (m"), s'effectuant à la température finale de congélation qui est inférieure à la température d'acclimatation ;
(n) une lyophilisation en deux étapes principales, dites primaire, (n'), et secondaire, (n") :
   - l'étape de lyophilisation primaire (n') s'effectuant par application d'un vide à environ 200 microbars et d'un profil de température ascendant ;
   - l'étape de lyophilisation secondaire (n") s'effectuant par application d'un vide à environ 50 microbars et d'un profil de température descendant.

Dans un mode de réalisation, le procédé selon l'invention est caractérisé en ce qu'il comporte en outre une étape de stérilisation du broyat de gelée de Wharton viro-inactivée par irradiation gamma après l'étape c).

Dans un mode de réalisation, le procédé selon l'invention est caractérisé en ce qu'il comporte en outre une étape de stérilisation du broyat de gelée de Wharton viro-inactivée par exposition de ce dernier à des rayonnements gamma à 25-32 kGy après l'étape c).

Dans un mode de réalisation, le procédé selon l'invention est caractérisé en ce qu'il comporte en outre une étape de stérilisation du broyat de gelée de Wharton viro-inactivée et lyophilisée par irradiation gamma après l'étape d).

Dans un mode de réalisation, le procédé selon l'invention est caractérisé en ce qu'il comporte en outre une étape de stérilisation du broyat de gelée de Wharton viro-inactivée et lyophilisée par exposition de ce dernier à des rayonnements gamma à 25-32 kGy après l'étape d).

L'utilisation de la composition comprenant un broyat de gelée de Wharton viro-inactivée est ci-après décrite. L'utilisation de la composition comprenant un broyat de gelée de Wharton, viro-inactivée et lyophilisée est également décrite.

L'utilisation d'une composition, ou d'une composition susceptible d'être obtenue selon le procédé ou d'un lyophilisat moulé tels que définis précédemment, dans le traitement d'une lésion tissulaire. est décrite.

L'utilisation d'une composition, ou d'une composition susceptible d'être obtenue selon le procédé ou d'un lyophilisat moulé tels que définis précédemment, dans le traitement d'une lésion cutanée ou dans le traitement d'une lésion oculaire est également décrite.

L'utilisation d'une composition, ou d'une composition susceptible d'être obtenue selon le procédé ou d'un lyophilisat moulé tels que définis précédemment, dans le comblement osseux ou le comblement d'une alvéole dentaire est également décrite.

L'utilisation du biomatériau ainsi que de la la composition comprenant au moins le biomatériau selon l'invention sont également décrites.

Dans un mode de réalisation, est décrite l'utilisation d'une composition, ou d'une composition susceptible d'être obtenue selon le procédé ou d'un lyophilisat moulé tels que définis précédemment, dans le traitement d'une lésion cutanée. Ladite lésion cutanée appartient au groupe comprenant : les inflammations cutanées aigues ou chroniques allergiques, les infections cutanées, les ulcères variqueux ou diabétiques, les brûlures cutanées, les plaies cutanées, les plaies de cicatrisation cutanées.

Dans un mode de réalisation, est décrite également l'utilisation d'une composition, ou d'une composition susceptible d'être obtenue selon le procédé ou d'un lyophilisat moulé tels que définis précédemment, dans le traitement d'une lésion oculaire. Ladite lésion oculaire appartient au groupe comprenant : les inflammations ou infections de la surface oculaire, le syndrome de l'œil sec, l'ulcère.

Dans un mode de réalisation, est décrite également l'utilisation d'une composition, ou d'une composition susceptible d'être obtenue selon le procédé ou d'un lyophilisat moulé tels que définis précédemment, dans le comblement osseux. Ledit comblement osseux appartient au groupe comprenant : le comblement d'une perte osseuse consécutive à une trépanation, le comblement d'une perte osseuse consécutive à une exérèse d'un fragment d'os, le comblement d'une perte osseuse consécutive à une ostéoporose, le comblement d'une perte osseuse consécutive au forage d'une cavité dans un os, le comblement d'une perte osseuse liée à un traumatisme, le comblement d'une perte osseuse liée à une fracture, le comblement d'une perte osseuse liée à une tumeur, le comblement lors d'une arthroplastie primaire ou secondaire, le comblement osseux dans le cadre d'une arthrodèse.

Dans un mode de réalisation, est décrite également l'utilisation d'une composition, ou d'une composition susceptible d'être obtenue selon le procédé ou d'un lyophilisat moulé tels que définis précédemment, dans le comblement d'une alvéole dentaire. Ladite alvéole dentaire nécessitant un comblement est consécutive à la perte de la dent normalement associée à ladite alvéole dentaire. La perte de la dent, nécessitant un comblement de l'alvéole dentaire, peut être due aux afflictions appartenant au groupe comprenant : perte d'une dent faisant suite à un traumatisme, perte d'une dent faisant suite à la pratique d'une extraction dentaire, perte d'une dent faisant suite à une infection, perte d'une dent faisant suite à une parodontite.

Dans un mode de réalisation, est décrite également également l'utilisation d'une composition, ou d'une composition susceptible d'être obtenue selon le procédé ou d'un lyophilisat moulé tels que définis précédemment, dans l'amélioration de la cicatrisation, la réduction de l'inflammation, l'amélioration de l'hydratation par des propriétés hygroscopiques.

Dans un mode de réalisation, l'utilisation est caractérisée en ce que la composition ou du lyophilisat moulé est utilisé sans réhydratation préalable.

Lorsque la composition ou le lyophilisat moulé est utilisé sans réhydratation préalable, la réhydratation peut s'effectuer in situ par hydratation naturelle par un fluide issu du corps du patient.

Dans un mode de réalisation, l'utilisation est caractérisée en ce que la composition ou du lyophilisat moulé est utilisé après réhydratation préalable.

Dans les applications sous forme de lyophilisat moulé avec principe actif, la composition selon l'invention peut être avantageusement positionnée lors du geste chirurgical *in situ* pour une diffusion locale (par exemples abcès ou tumeur intra-osseux, intracérébral) compte tenu de sa manipulation aisée.

La demanderesse a également mis en évidence l'intérêt de l'invention comme facteur d'enrobage dans la bio-impression. En effet, la composition, ou la composition susceptible d'être obtenue selon le procédé, ou le lyophilisat moulé tels que définis précédemment, présente plusieurs caractéristiques avantageuses pour ce type d'utilisation.

En effet, l'environnement biologique de la composition en tant que matrice extracellulaire (notamment en glycosaminoglycanes, protéoglycanes, collagènes) ; ainsi que les facteurs de croissance qu'elle comprend (notamment FGF, IGF-I, TGF, PDGF, EGF), font de la composition un vecteur idéal pour enrober les biomatériaux et/ou cellules destinées à la bio-impression.

De plus, la composition selon l'invention est stable à la chaleur dans ses caractéristiques. Certains matériaux utilisés en bio-impression nécessitant d'être chauffés et refroidis avant bio-impression, la composition selon l'invention permet donc de favoriser les échanges thermiques entre ces matériaux et le milieu extérieur.

De fait, la composition selon l'invention est idéale comme facteur d'enrobage dans la bio-impression pour entrer dans la composition des bioencres.

Dans un mode de réalisation, l'invention concerne également l'utilisation d'une composition, ou d'une composition susceptible d'être obtenue selon le procédé ou d'un lyophilisat moulé tels que définis précédemment, dans la bio-impression.

Dans un mode de réalisation, l'invention concerne également l'utilisation d'une composition, ou d'une composition susceptible d'être obtenue selon le procédé ou d'un lyophilisat moulé tels que définis précédemment, en tant que facteur d'enrobage à l'impression dans la bio-impression.

Des méthodes de traitement d'une maladie ou condition par administration de la composition comprenant un broyat de gelée de Wharton viro-inactivée sont ci-après décrites. Des méthodes de traitement d'une maladie ou condition par administration de la composition comprenant un broyat de gelée de Wharton, viro-inactivée et lyophilisée sont également décrites.

Il est donc décrit une méthode pour traiter une lésion tissulaire, caractérisée en ce qu'elle comprend l'administration topique d'une quantité thérapeutiquement suffisante d'une composition, ou d'une composition susceptible d'être obtenue selon le procédé ou d'un lyophilisat moulé tels que définis précédemment, à un patient.

Il est donc décrit une méthode pour traiter une lésion cutanée ou une lésion oculaire, caractérisée en ce qu'elle comprend l'administration topique d'une quantité thérapeutiquement suffisante d'une composition, ou d'une composition susceptible d'être obtenue selon le procédé ou d'un lyophilisat moulé tels que définis précédemment, à un patient.

Dans un mode de réalisation, il est décrit une méthode pour traiter une lésion cutanée appartenant au groupe comprenant les inflammations cutanées aigues ou chroniques allergiques, les infections cutanées, les ulcères variqueux ou diabétiques, les brûlures cutanées, les plaies cutanées, les plaies de cicatrisation cutanées, caractérisée en ce qu'elle comprend l'administration topique d'une quantité thérapeutiquement suffisante d'une composition, ou d'une composition susceptible d'être obtenue selon le procédé ou d'un lyophilisat moulé tels que définis précédemment, à un patient.

Dans un mode de réalisation, il est décrit une méthode pour traiter une lésion oculaire appartenant au groupe comprenant les inflammations ou infections de la surface oculaire, le syndrome de l'œil sec, l'ulcère, caractérisée en ce qu'elle comprend l'administration topique d'une quantité thérapeutiquement suffisante d'une composition, ou d'une composition susceptible d'être obtenue selon le procédé ou d'un lyophilisat moulé tels que définis précédemment, à un patient.

Il est décrit également une méthode de comblement osseux, caractérisée en ce qu'elle comprend l'administration d'une quantité thérapeutiquement suffisante d'une composition, ou d'une composition susceptible d'être obtenue selon le procédé ou d'un lyophilisat moulé tels que définis précédemment, à un patient.

Il est décrit également une méthode de comblement pour traiter une perte osseuse, caractérisée en ce qu'elle comprend l'administration d'une quantité thérapeutiquement suffisante d'une composition, ou d'une composition susceptible d'être obtenue selon le procédé ou d'un lyophilisat moulé tels que définis précédemment, à un patient.

Pour le traitement d'une perte osseuse, cette administration d'une quantité thérapeutiquement suffisante peut être définie comme étant celle au moins nécessaire pour réaliser le comblement osseux. Ladite perte osseuse appartient au groupe comprenant : la perte osseuse consécutive à une trépanation, la perte osseuse consécutive à une exérèse d'un fragment d'os, la perte osseuse consécutive à une ostéoporose, la perte osseuse consécutive au forage d'une cavité dans un os, la perte osseuse liée à un traumatisme, la perte osseuse liée à une fracture, la perte osseuse liée à une tumeur.

Il est décrit également une méthode de comblement pour bloquer ou fusionner une articulation lors de la réalisation d'une arthrodèse, caractérisée en ce qu'elle comprend l'administration d'une quantité thérapeutiquement suffisante d'une composition, ou d'une composition susceptible d'être obtenue selon le procédé ou d'un lyophilisat moulé tels que définis précédemment, à un patient.

Il est décrit également une méthode de comblement pour remplacer une articulation lors de la réalisation d'une arthroplastie primaire ou secondaire, caractérisée en ce qu'elle comprend l'administration d'une quantité thérapeutiquement suffisante d'une composition, ou d'une composition susceptible d'être obtenue selon le procédé ou d'un lyophilisat moulé tels que définis précédemment, à un patient.

Il est décrit également une méthode pour traiter une alvéole dentaire, caractérisée en ce qu'elle comprend l'administration d'une quantité thérapeutiquement suffisante d'une composition, ou d'une composition susceptible d'être obtenue selon le procédé ou d'un lyophilisat moulé tels que définis précédemment, à un patient.

Pour le traitement d'une alvéole dentaire, il est considéré que celle-ci est vacante de la dent naturellement associée, suite à un traumatisme ou une maladie tels que précédemment décrits.

Ladite alvéole dentaire nécessitant un comblement est consécutive à la perte de la dent normalement associée à ladite alvéole dentaire. La perte de la dent, nécessitant un comblement de l'alvéole dentaire, peut être due aux afflictions appartenant au groupe comprenant : perte d'une dent faisant suite à un traumatisme, perte d'une dent faisant suite à la pratique d'une extraction dentaire, perte d'une dent faisant suite à une infection, perte d'une dent faisant suite à une parodontite.

Pour le traitement d'une alvéole dentaire, cette administration d'une quantité thérapeutiquement suffisante peut être définie comme étant celle au moins nécessaire pour réaliser le comblement de ladite alvéole dentaire.

Dans un mode de réalisation, il est décrit également une méthode pour améliorer la cicatrisation, la réduction de l'inflammation, l'amélioration de l'hydratation par des propriétés hygroscopiques, caractérisée en ce qu'elle comprend l'administration topique d'une quantité thérapeutiquement suffisante d'une composition, ou d'une composition susceptible d'être obtenue selon le procédé ou d'un lyophilisat moulé tels que définis précédemment, à un patient.

Dans un mode de réalisation, les méthodes précédemment décrites sont caractérisées en ce que l'administration topique d'une quantité thérapeutiquement suffisante de la composition ou du lyophilisat moulé est effectuée sans réhydratation préalable de la composition ou du lyophilisat moulé.

Dans un mode de réalisation, les méthodes précédemment décrites sont caractérisées en ce que l'administration topique d'une quantité thérapeutiquement suffisante de la composition ou du lyophilisat moulé est effectuée après réhydratation préalable de la composition ou du lyophilisat moulé.

Dans un mode de réalisation, les méthodes de comblement osseux et pour traiter une alvéole dentaire sont caractérisées en ce que l'administration d'une quantité thérapeutiquement suffisante de la composition ou du lyophilisat moulé est effectuée sans réhydratation préalable de la composition ou du lyophilisat moulé.

Dans un mode de réalisation, les méthodes de comblement osseux et pour traiter une alvéole dentaire sont caractérisées en ce que l'administration d'une quantité thérapeutiquement suffisante de la composition ou du lyophilisat moulé est effectuée après réhydratation préalable de la composition ou du lyophilisat moulé.

Des méthodes de bio-impression par insertion de biomatériaux ou cellules destinés à être bio-imprimés dans la composition comprenant un broyat de gelée de Wharton viro-inactivée sont ci-après décrites. Des méthodes de bio-impression par insertion de biomatériaux ou cellules destinés à être bio-imprimés dans la composition comprenant un broyat de gelée de Wharton viro-inactivée et lyophilisée sont ci-après décrites.

L'invention concerne donc également une méthode pour enrober des matériaux ou des cellules destinés à la bio-impression, caractérisée en ce qu'elle comprend les étapes suivantes :
- on introduit dans la composition, ou dans la composition susceptible d'être obtenue selon le procédé ou d'un lyophilisat moulé tels que définis précédemment, les biomatériaux ou les cellules destinés à être bio-imprimés ;
- on homogénéise le mélange précédemment obtenu afin d'obtenir une bioencre ;
- on introduit la bioencre dans la cartouche de la bio-imprimante.

Dans cette application, par exemple la composition selon l'invention lyophilisée irradiée sous la forme d'un disque adapté au réceptacle de bio-encre de l'imprimante 3 D sera réhydratée par une solution cellulaire avec le milieu de culture, qui sera déposée dans le réceptacle contenant la gelée de Wharton, pour une reformation du gel qui enchâssera les cellules et assurera une reconstitution du gel.

Ce gel cellulaire (cellules et gelée de Wharton) sera la matière d'échafaudage du tissu. L'imprimante fera donc tomber une goutte de gel cellulaire composé d'une ou 2 cellules, une fibre collagène et des GAG enrobant ces cellules.

L'organisation des cellules en sera facilitée, comme pour le derme puisque les cellules cohabitent avec la matrice extracellulaire physiologique. Les connections cellulaires et l'attachement cellulaire du réseau fibrillaire sont donc facilités.

La matrice extracellulaire est une structure importante pour que les cellules puissent s'organiser dans une imprimante 3D. Un lacis de microfibres (fibres de collagènes de la gelée de Wharton) dans lequel les cellules peuvent s'attacher et avoir un réseau de glissement naturel (par les GAG sulfatés et non sulfatés) pour communiquer par l'intermédiaire d'un gel qui enrobe et qui facilite la communication intercellulaire et la mise en œuvre d'un échafaudage.

Le derme cellulaire est composé d'une matrice extracellulaire similaire à la gelée de Wharton.

Les cellules dans le milieu de culture sont le support de réhydratation de la gelée de Wharton lyophilisée, les cellules sont alors à la fois en contact avec le microréseau de collagène et enrobées par les GAG de la gelée de Wharton, c'est cette solution qui est alors l'encre biologique qui est donc utilisée pour la reconstitution des vaisseaux.

L'association du gel avec d'autres gels qui doivent être parfois chauffés pour obtenir leurs dissolutions ou leurs formes liquides, puis refroidis pour pouvoir être mélangé aux cellules. Le mélange de gelée de Wharton réfrigérée permet de réguler rapidement les gels chauffés et aussi protéger les cellules au moment de la préparation.

### EXEMPLES

### Exemple 1 : Exemple d'un mode de réalisation de l'invention selon lequel un segment de gelée de Wharton est traité selon le procédé pour obtenir une composition utilisable dans le traitement de l'œil (par exemple conjonctivite ou syndrome de l'œil sec).

Un donneur proprement informé et consentant en accord avec les exigences de la Déclaration d'Helsinki offre en don le cordon ombilical issu d'un accouchement. Par exigence sanitaire relative aux dons de tissus et de cellules d'origine humaine, une qualification en amont du donneur est systématique. Cette qualification implique une recherche de virus HIV, hépatites B, C, HTLV et de la bactérie tréponème pâle responsable de la syphilis.

Le cordon ombilical est récupéré le plus tôt possible en salle d'accouchement. Il est avantageusement placé dans une boite stérile, comprenant une solution de NaCl à +4°C.

Au laboratoire, en salle stérile, la procédure suivante est appliquée :

Un segment de 20 cm à 50 cm de longueur est isolé par section du cordon ombilical.

Le cordon ombilical est rincé et hydraté dans des bains d'eau purifiée successifs, sous agitation douce, pendant 4 heures.

Les vaisseaux sanguins du segment de cordon ombilical sont identifiés et séparés du reste du segment afin de conserver uniquement la gelée de Wharton et la membrane amniotique qui l'entoure. La gelée de Wharton et la membrane amniotique sont utilisées dans la suite du procédé sous l'appellation générale de gelée de Wharton, car la quantité en poids de membrane est négligeable par rapport à la quantité en poids de gelée de Wharton.

La gelée de Wharton est congelée à sec à une température de -80°C.

Lors du traitement chimique, la gelée de Wharton est décongelée à l'air libre, à température ambiante, pendant une durée de 5 minutes. Cette étape de congélation, suivie d'une décongélation, assure une décellularisation importante du tissu biologique.

La gelée de Wharton subit une succession de bains assurant un traitement chimique de celle-ci. Ce traitement a pour objectif une désinfection de la gelée de Wharton, et tout particulièrement sa viro-inactivation. Une agitation linéaire douce du milieu liquide est appliquée lors de chaque bain afin d'assurer une pénétration homogène des solvants dans les tissus.

Dans un premier temps, la gelée de Wharton est déposée dans un bain d'eau purifiée à température ambiante pendant environ 3 heures. Cette étape assure tant la fin de la décongélation du tissu physiologique, qu'une étape de lyse cellulaire par pression osmotique.

Puis, la gelée de Wharton est transférée dans un bain décontaminant composé d'éthanol 70 % v/v à température ambiante pendant environ 1 heure.

Un lavage est effectué dans de l'eau purifiée pendant environ 15 minutes à température ambiante pour retirer l'éthanol.

Afin d'assurer la seconde étape de traitement décontaminant, la gelée de Wharton est transférée dans un bain composé de peroxyde d'hydrogène à 30 % w/v à température ambiante pendant environ 15 minutes.

Puis, la gelée de Wharton est transférée dans un bain décontaminant composé de peroxyde d'hydrogène à 3 % w/v à température ambiante pendant environ 1 heure. La gelée de Wharton obtenue est viro-inactivée.

L'action chimique appliquée à la gelée de Wharton viro-inactivée est ensuite neutralisée, dans un bain comportant de l'hydroxyde de sodium dilué autour d'un pH de 8,5. Le traitement du bain de neutralisation s'effectue à température ambiante pendant environ 15 minutes.

Afin d'assurer un rééquilibrage du pH et pour éliminer au mieux les résidus organiques se détachant du tissu d'intérêt, la gelée de Wharton viro-inactivée est transférée dans un bain de tampon physiologique (PBS), afin d'assurer son rééquilibrage physiologique. Le bain s'effectue à température ambiante pendant environ 15 minutes.

Enfin, la gelée de Wharton est transférée dans deux bains successifs à l'eau purifiée, à température ambiante, pendant au moins 15 minutes et jusqu'à environ 1 heure.

A cette étape du procédé, on peut considérer que la gelée de Wharton obtenue selon ce traitement chimique est une gelée de Wharton en grande partie désinfectée, notamment viro-inactivée, et décellularisée.

Suite à cette partie relative aux traitements chimiques, la gelée de Wharton viro-inactivée subit une étape de broyage.

La gelée de Wharton est insérée dans un broyeur centrifuge Recht Z200, équipé avec un tamis dont la maille est de 3 mm. Un broyage de moins de 1 minute est effectué à une vitesse de rotation de 14000 tours/minute et la substance est récupérée. Un deuxième broyage avec un tamis dont la maille est de 1 mm est ensuite effectué sur une durée de moins de 1 minute à une vitesse de rotation de 14000 tours/minute. La substance obtenue est une substance liquide gélifiée homogène. Cette substance est mise dans un flacon.

Pour pouvoir utiliser la gelée de Wharton en tant que vecteur de principe actif destiné au traitement des conjonctivites, de la gentamicine est mélangée au broyat de gelée de Wharton viro-inactivé. Une solution de gentamicine est introduite et mélangée au broyat de gelée de Wharton viro-inactivé de telle sorte que la teneur massique finale en gentamicine dans le produit soit de 3%.

A cette étape du procédé, la gelée de Wharton viro-inactivée et broyée est mise en forme dans un moule. Ce moule accompagne idéalement la composition tout au long de son cycle de vie, de l'étape de mise en forme jusqu'à la mise à disposition de l'utilisateur.

Le moule utilisé est plusieurs réceptacles en Polyéthylène (PE) ou en Polyéthylène téréphtalate glycolisé (PETG) de forme cylindrique dont le diamètre mesure 0,4 cm et dont la hauteur mesure également 0,4 cm.

La gelée de Wharton viro-inactivée et broyée est mise en forme dans autant de moules que désirés et que la quantité de gelée de Wharton viro-inactivée et broyée permet. Une pipette de 1 mL est avantageusement utilisée pour mettre en forme la gelée de Wharton viro-inactivée et broyée dans les moules.

Suite à cette partie relative au broyage et à la mise en forme, la gelée de Wharton viro-inactivée et broyée subit une lyophilisation.

La gelée de Wharton viro-inactivée et broyée qui est contenue dans le moule est placée sur un plateau en inox.

L'ensemble est transféré dans un lyophilisateur, où une étape de congélation suivie d'une étape de lyophilisation sont effectuées selon les modalités suivantes :
- Congélation :
   - La première étape de congélation s'effectue à une température d'acclimatation choisie pour ne pas endommager l'intégrité structurelle, fonctionnelle et biologique de la gelée de Wharton viro-inactivée et broyée ;
   - la deuxième étape de congélation s'effectue à la température finale de congélation qui est inférieure à la température d'acclimatation ;
- Lyophilisation :
   - Une étape de lyophilisation primaire s'effectue par application d'un vide à environ 200 microbars et par application d'un profil de température ascendant ;
   - Une étape secondaire de lyophilisation s'effectue par application d'un vide à environ 50 microbars et par application d'un profil de température descendant.

Suite à cette étape de lyophilisation, le produit obtenu est un broyat de gelée de Wharton, désinfectée, notamment viro-inactivée, décellularisée et lyophilisée. De plus, le produit obtenu est défini par une forme de cylindre d'un diamètre de 0,4 cm et d'une hauteur de 0,3 cm, qui est conférée par le moule suite à l'étape de lyophilisation.

Chacun des broyats de gelée de Wharton, viro-inactivée, décellularisée et lyophilisée ainsi formés est détaché aisément de son moule et repositionné dans le même moule et mis dans un conditionnement primaire qui est un sachet TYVEK^{®} en copolymère PE-PET.

Une dernière étape de stérilisation du broyat de gelée de Wharton, viro-inactivée, décellularisée et lyophilisée est effectuée par exposition de ce broyat à des rayonnements gamma à 25-32 kGrays. Tous les sachets comprenant les broyats obtenus à partir de la substance biologique initiale sont traités simultanément lors de cette étape de stérilisation par rayonnements gamma.

Le produit ainsi obtenu est utilisé comme composition pour application topique.

Quelques utilisations envisageables du produit obtenu selon le procédé sont par exemples le traitement d'une conjonctivite, du syndrome de l'œil sec, ou le traitement d'une lésion cutanée comme cicatrisant.

Lorsque le produit est utilisé dans le traitement d'une conjonctivite, la méthode consiste en une réhydratation in situ du broyat de gelée de Wharton, viro-inactivée, décellularisée et lyophilisée, puis le broyat est massé par l'hydratation de l'œil.

### Exemple 2 : Démonstration de l'efficacité de la viro-inactivation d'un produit biologique placentaire selon le procédé.

La demanderesse a démontré l'efficacité de la viro-inactivation chimique selon l'invention sur des produits biologiques issus du placenta qui sont des membranes chorioamniotiques

Des mesures de la charge virale ont été effectuées dans des membranes chorioamniotiques, par injection de virus entre l'amnios et le chorion afin de vérifier la viro-inactivation des tissus selon le procédé.

Des aliquotes des membranes ainsi traitées sont prélevées afin d'être mises en solution et centrifugées. La fraction soluble sert à ensemencer des cellules en milieu de culture cellulaire. Chaque type de virus étudié est ensemencé sur le type adéquat de cellules afin de permettre l'expression de sa virulence.

Les virus ayant fait l'objet d'une mesure sont le virus Parvovirus porcin (ci-après noté PPV), le virus Pseudorabies (ci-après noté PRV) impliqué dans la maladie d'Aujeszky, et le virus de l'immunodéficience humaine de type 1 (ci-après noté HIV-1), le virus de l'hépatite A (ci-après noté HAV).

Les mesures de charge virale ont été effectuées par détection de production virale dans des cellules infectées, et plus particulièrement par observation d'un effet spécifique cytopathique. Les virus injectés dans les couches membranaires ont subi soit l'étape de traitement chimique à l'éthanol à 70% v/v selon le procédé, soit l'étape de traitement chimique au peroxyde d'hydrogène à 30% w/v puis 3% w/v selon le procédé.

Chaque mesure de la charge virale a été effectuée deux fois pour chaque essai, celles-ci sont notées dans le tableau suivant par les indications Run A et Run B.

Les résultats dans le tableau suivant représentent la diminution de la charge virale mesurée, exprimée en log. Cette diminution est mesurée par comparaison de la charge virale sur la couche membranaire avant, puis après le traitement indiqué.

| **Résultats** | | |
|---|---|---|
| **Virus** | **Traitement H₂O₂** | **Traitement Ethanol** |
| **PPV** | Run A = 4,53 | Run A = 1,43 |
| | Run B = 3,94 | Run B = 2,03 |
| **PRV** | Run A > 4,47 | Run A > 5,18 |
| | Run B > 4,47 | Run B > 4,83 |
| **HAV** | Run A > 4,47 | Run A = 1,37 |
| | Run B > 4,53 | Run B = 1,37 |
| **HIV-1** | Non applicable | Run A > 4,52 |
| | | Run B > 4,50 |

L'efficacité des traitements chimiques selon le procédé, appliqués aux couches membranaires, et destinés à la viro-inactivation peut être constatée.

Le traitement chimique au peroxyde d'hydrogène est efficace contre le PPV, le PRV et l'HAV. Concernant le HIV-1, les conditions de réalisation des mesures n'ont pas pu donner de résultats interprétables.

Le traitement chimique à l'éthanol est efficace contre le PRV et le HIV-1, et dans une moindre mesure efficace contre les virus nus (non enveloppés) comme le PPV et l'HAV.

Comme démontré, seule la combinaison des deux traitements chimiques permet d'assurer une viro-inactivation des couches membranaires, tant des virus enveloppés que des virus nus. Cette viro-inactivation étant définie par une diminution de la population virale d'au moins 4 log.

### Exemple 3 : Démonstration de l'efficacité de la viro-inactivation d'un produit biologique placentaire selon le procédé.

La demanderesse a démontré l'efficacité de la viro-inactivation chimique selon l'invention sur des produits biologiques issus du placenta qui sont des membranes amniotiques liées à leurs membranes tissulaires spongieuses.

Des mesures de la charge virale ont été effectuées dans des membranes amniotiques dont la liaison avec leurs membranes tissulaires spongieuses a été préservée, par injection de virus entre la membrane amniotique et la membrane tissulaire spongieuse afin de vérifier la viro-inactivation des tissus selon le procédé.

Des aliquotes des membranes ainsi traitées sont prélevées afin d'être mises en solution et centrifugées. La fraction soluble sert à ensemencer des cellules en milieu de culture cellulaire. Chaque type de virus étudié est ensemencé sur le type adéquat de cellules afin de permettre l'expression de sa virulence.

Les virus ayant fait l'objet d'une mesure sont le virus Parvovirus porcin (ci-après noté PPV) et le virus de l'immunodéficience humaine de type 1 (ci-après noté HIV-1).

Les mesures de charge virale ont été effectuées par détection de production virale dans des cellules infectées, et plus particulièrement par observation d'un effet spécifique cytopathique. Les virus injectés dans les couches membranaires ont subi l'étape de traitement chimique au peroxyde d'hydrogène à 30% w/v puis 3% w/v selon le procédé.

Chaque mesure de la charge virale a été effectuée deux fois pour chaque essai, celles-ci sont notées dans le tableau suivant par les indications Run A et Run B.

Les résultats dans le tableau suivant représentent la diminution de la charge virale mesurée, exprimée en log. Cette diminution est mesurée par comparaison de la charge virale sur la couche membranaire avant, puis après le traitement indiqué.

| **Résultats** | |
|---|---|
| **Virus** | **Traitement H₂O₂** |
| **HIV-1** | Run A > 6,02 |
| | Run B > 5,90 |
| **PPV** | Run A = 6,47 |
| | Run B = 5,66 |

L'efficacité de l'étape de traitement chimique au peroxyde d'hydrogène à 30% w/v puis 3% w/v selon le procédé, appliquée aux couches membranaires, et destinée à la viro-inactivation peut être constatée.

Le traitement chimique au peroxyde d'hydrogène, appliqué à une membrane amniotique dont la liaison avec leurs membranes tissulaires spongieuses a été préservée, est efficace contre le PPV et le HIV-1.

Comme démontré, le traitement au peroxyde d'hydrogène selon le procédé permet d'assurer une viro-inactivation des couches membranaires envers le virus HIV-1 et le virus PPV. Cette viro-inactivation étant définie par une diminution de la population virale d'au moins 4 log.

### Exemple 4 : Mise en évidence de la préservation des qualités structurelles de la composition selon l'invention.

L'objectif des essais est de déterminer la consistance du broyat de gelée de Wharton en fonction de l'étape de broyage réalisée avant ou après lyophilisation. La consistance de la composition est évaluée après réhydratation. Le but est de déterminer si la même consistance est obtenue ou non.

Trois échantillons de cordons ombilicaux (Lot 1, Lot 2 et Lot 3) sont réceptionnés, préparés et viro-inactivés chimiquement selon les mêmes étapes que décrites dans l'Exemple 1. Puis chacun des lots subit les étapes de traitement suivants :
Lot 1 :
A la fin du traitement chimique, le tissu du Lot 1 est broyé dans un broyeur centrifuge avec un tamis dont la maille est de 3 mm à une vitesse de 14000 tours/minute pendant moins de 1 minute.

Le broyat obtenu est récupéré à l'aide d'une maryse et transféré en salle de lyophilisation dans un flacon de 50 mL.

Le broyat de gelée de Wharton est réparti en 7 flacons de 7 mL de façon à avoir la moitié du flacon remplie. Les flacons sont positionnés dans le lyophilisateur avec un bouchon. Ces flacons ne sont pas bouchés sous vide, le bouchon permet juste de faire barrière avec l'extérieur.

Une étape de lyophilisation est effectuée telle que décrite dans l'Exemple 1.

Après lyophilisation, la hauteur de liquide a peu diminué. Les bouchons sont fermés après la lyophilisation. Certaines gelées se décollent de la paroi et se rétractent.

Le broyat de gelée de Wharton viro-inactivée et lyophilisée contenue dans l'un des 7 flacons est ensuite réhydratée. La masse de la gelée lyophilisée avant réhydratation est de 469,8 mg. Pour réhydrater totalement le produit, 2100 µL d'eau distillée sont ajoutés. Le produit une fois hydraté a la même consistance qu'avant lyophilisation.

Lot 2 et Lot 3 :
A la fin du traitement chimique, les tissus du Lot 2 et du Lot 3 sont lyophilisés sans que les tissus biologiques ne soient broyés.

Les tissus biologiques sont donc des bandes de tissu de gelée de Wharton qui sont lyophilisées directement sans mise en forme (pas de découpe, ni broyage). Quatre à cinq bandes de tissu biologique de 5 à 15 centimètres chacun sont utilisées pour chaque essai.

Le tissu du Lot 2 est déposé sur un filtre d'autoclavage avec la membrane amniotique de la gelée de Wharton du côté du filtre.

Le tissu du Lot 3 est déposé sur le filtre avec le côté qui ne comprend pas la membrane amniotique sur le filtre. Un deuxième filtre est posé par-dessus (donc au contact de la membrane amniotique).

La comparaison des essais sur les lots Lot 2 et Lot 3 permet d'estimer l'impact d'un plus faible échange entre la membrane amniotique de la gelée de Wharton et l'étagère du lyophilisateur.

Une étape de lyophilisation est réalisée telle que décrite dans l'Exemple 1.

Après lyophilisation, aucune différence entre les deux types de configuration n'est observée.

La gelée de Wharton lyophilisée des Lots 2 et 3 obtenue est ensuite broyée. Trois outils de broyage sont évalués, le Lot 2 et le Lot 3 sont donc répartis en quantités identiques en trois échantillons.

Les trois types de broyeurs testés sont : un broyeur centrifuge (selon les mêmes paramètres que pour le Lot 1), un broyeur à couteaux, un blender PC 250.

Résultat observé avec le broyeur centrifuge : les bandes s'envolent et se bloquent dans le broyeur, les fragments brûlent car se bloquent dans l'outil. Le produit obtenu est donc constitué de fibres assez fines dont certaines sont colorées par la combustion.

Résultats observés avec le broyeur à couteaux (marque Essentiel B) et le blender PC 250 : le broyage présente des difficultés car les lames sont assez écartées du fond. Les morceaux de gelée de Wharton lyophilisés sont électrostatiques, adhérent donc aux parois et ne sont donc pas découpés. Cependant le blender broie plus finement que le broyeur à couteaux.

Les produits obtenus (environ 1 cm³ par essai pour le Lot 2 et le Lot 3) sont réhydratés avec 150 µL d'eau distillée. Un gel filamenteux cohésif est obtenu. Les produits obtenus à partir des lots Lot 2 et Lot 3 sont macroscopiquement comparés à la gelée de Wharton broyée avant lyophilisation puis réhydratée (Lot 1). La consistance de ces gels est proche de la gelée du Lot 1 avant lyophilisation hormis une présence de filaments.

Le broyage après lyophilisation est très compliqué du fait de la légèreté, de la forme et des propriétés électrostatiques de la gelée de Wharton lyophilisée. Le produit est également très sensible à l'humidité : un phénomène de rétraction du produit après réhydratation est observé.

Le broyage avant lyophilisation donne un liquide visqueux qui a la texture d'un gel. Une fois lyophilisée, la gelée broyée a la texture d'une mousse et peut être réhydratée pour retrouver la même consistance que le gel obtenu avant lyophilisation.

Ce broyage avant l'étape de lyophilisation par un broyeur centrifuge est donc particulièrement adapté pour l'obtention d'un gel à partir de gelée de Wharton traitée.

Ce broyage avant l'étape de lyophilisation par un broyeur centrifuge est particulièrement adapté pour l'obtention d'un gel à partir de gelée de Wharton traitée qui soit dépourvue de fibres de collagène épaisses et/ou de parois ou lacunes et parois vasculaires.

### Exemple 5 : Exemples de mise en forme du broyat de gelée de Wharton viro-inactivée et lyophilisée selon l'invention

L'objectif des essais est de tester différentes méthodes de mise en forme de la gelée de Wharton traitée et broyée de manière à déterminer les possibilités de mise en forme de cette dernière. Différents tamis de broyage sont également testés pour évaluer l'impact sur le produit fini.

Des échantillons de cordons ombilicaux sont réceptionnés, préparés et viro-inactivés chimiquement selon les mêmes étapes que décrites dans l'Exemple 1.

### Protocole :

L'étape de broyage est ensuite effectuée en faisant varier le paramètre de la taille de maille du tamis utilisé dans le broyeur centrifuge : une gelée de Wharton viro-inactivée est broyée avec le tamis de 1 mm (Lot 4), une autre avec le tamis de 3 mm (Lot 5) et la troisième est broyée avec le tamis de 3 mm puis avec le tamis de 1 mm (Lot 6).

Les broyats de gelée de Wharton obtenus sont récupérés à l'aide d'une maryse et transférés en salle de lyophilisation dans des flacons de 50 mL.

Les broyats de gelée de Wharton sont ensuite répartis dans les plaques avec différentes tailles de puits. Un broyat de gelée de Wharton est réparti dans une plaque 96 puits. Un autre dans une plaque 48 puits et le troisième est réparti dans des plaques de 24 puits, 12 puits et 6 puits. Cette répartition est effectuée à l'aide d'une seringue de 2,5 mL et d'une pipette de 10,0 mL. La plaque 96 puits est remplie à la seringue et toutes les autres avec la pipette. Différents volumes de remplissage sont testés pour créer des produits d'épaisseurs différentes.

Les couvercles des plaques contenant les broyats sont mis sur ces dernières mais pas dans l'axe de manière à couvrir les broyats sans complétement fermer les plaques. Sur la plaque 96 puits, une partie des broyats est laissée non couverte par le couvercle pour déterminer si une différence se produit entre le broyat couvert et le broyat non couvert. Cette différence est évaluée macroscopiquement.

Les broyats de gelée de Wharton ainsi répartis sont ensuite lyophilisés, le même protocole de lyophilisation tel que décrit l'Exemple 1 est utilisé.

Une fois la lyophilisation terminée les broyats de gelée de Wharton viro-inactivée et lyophilisée sont récupérés à l'aide d'une spatule en inox et d'une pince inox fine courbe. Le diamètre et l'épaisseur des broyats de gelée de Wharton viro-inactivée et lyophilisée sont mesurés à l'aide d'un réglet en inox.

Un échantillon de broyat de gelée de Wharton viro-inactivée et lyophilisée est placé dans un récipient et réhydraté à l'aide d'une pipette et d'eau purifiée jusqu'à la limite de saturation de la gelée en eau. Une spatule en inox est utilisée pour mélanger la gelée lyophilisée avec l'eau. Après réhydratation le volume de gel obtenu est mesuré.

Un autre échantillon est beaucoup plus hydraté avec un volume d'eau supérieur à la saturation pour bien séparer les fibres les unes des autres.

Une petite quantité de ces broyats réhydratés est prélevée et étalée sur un intercalaire pour visualiser la forme des morceaux présents dans les broyats réhydratés. Pour ce faire, une pince et une spatule sont utilisées pour bien séparer et étaler tous les constituants des broyats réhydratés.

### Résultats des broyages :

Le premier échantillon (Lot 4) est broyé avec le tamis de 1 mm. Après broyage, le broyat obtenu n'est pas broyé très finement, des morceaux filamenteux sont présents dans le broyat obtenu. Ce broyat obtenu est assez visqueux. Ce broyat de gelée de Wharton est sous forme de morceaux filamenteux.

Le broyat de gelée de Wharton est récupéré dans un flacon de 50 mL et envoyé en salle de lyophilisation où il est réparti dans une plaque 96 puits.

Le second échantillon (Lot 5) est broyé avec le tamis de 3 mm. Après broyage le broyat a la texture d'un gel très liquide. Le broyat semble contenir beaucoup d'eau ce qui expliquerait le fait que le broyat soit si liquide.

Le broyat de gelée de Wharton est récupéré dans un flacon de 50 mL et envoyé en salle de lyophilisation où il est réparti dans une plaque 48 puits.

Le troisième échantillon (Lot 6) est broyé avec le tamis de 3 mm et broyé une seconde fois avec le tamis de 1 mm. Le broyat de gelée de Wharton obtenu a la texture d'un gel. Le broyage avec le tamis de 1 mm a permis d'obtenir un meilleur résultat qu'avec le Lot 4.

Le broyat de gelée de Wharton est récupéré dans un flacon de 50 mL et envoyé en salle de lyophilisation où il est réparti dans des plaques de 24 puits, 12 puits et 6 puits.

Répartition dans les puits, préformage des produits moulés :

Les volumes de gelées mis dans les puits sont les suivants :

| Echantillon | Type de plaque | Volume de broyat par puit | Nombre de puits remplis |
|---|---|---|---|
| Lot 4 (tamis 1 mm) | 96 puits | 0,4 mL | 25 |
| | | 0,2 mL | 24 |
| Lot 5 (tamis 3 mm) | 48 puits | 0,4 mL | 24 |
| | | 0,8 mL | 9 |
| Lot 6 (tamis 3 puis 1 mm) | 24 puits | 1,0 mL | 3 |
| | | 0,8 mL | 6 |
| | | 0,5 mL | 2 |
| | 12 puits | 1,1 mL | 1 |
| | | 1,5 mL | 2 |
| | | 2,0 mL | 1 |
| | 6 puits | 25 mL | 2 |

Les aliquotes sont réalisés aisément à la seringue (Lot 4) ou à la pipette (Lot 5, Lot 6). La texture broyée permet une répartition fluide et ajustée des volumes.

### Résultats après lyophilisation :

La pince inox fine courbe permet une préhension aisée des broyats de gelée de Wharton viro-inactivée et lyophilisée, qui se présentent comme une mousse flexible.

Les produits ont l'aspect de mousses souples qui ont la forme des puits dans lesquels ils ont été lyophilisés.

Pour la plaque 96 puits, il n'y a pas de différences observées entre les broyats de gelée de Wharton viro-inactivée et lyophilisée couvertes par le couvercle et les non couvertes.

Les broyats de gelée de Wharton viro-inactivée et lyophilisée sont récupérés sous formes de disques et de cylindres. Les diamètres et épaisseurs mesurés sont les suivants :

| | | | Numéro d'échantillons et nombre de broyats lyophilisés récupérés | | |
|---|---|---|---|---|---|
| Type de plaque | Diamètre | Epaisseur | Lot 4 | Lot 5 | Lot 6 |
| 6 puits | 3,4 cm | 0,20 cm (0,4 cm sur les bords)^{∗} | / | 2 | / |
| 96 puits (0,4 mL) | 0,5 cm | 1,10 cm | 24 | / | / |
| 96 puits (0,2 ml) | 0,5 cm | 0,60 cm | 24 | / | / |
| 12 puits (1,1 mL) | 2,0 cm | 0,20 cm (0,40 cm sur les bords)^{∗} | / | 1 | / |
| 12 puits (2,0 mL) | 2,0 cm | 0,40 cm (0,70 cm sur les bords)^{∗} | / | 1 | / |
| 12 puits (1,5 mL) | 2,0 cm | 0,30 cm (0,50 cm sur les bords)^{∗} | / | 2 | / |
| 48 puits (0,4 mL) | 1,1 cm | 0,25 cm | / | / | 24 |
| 24 puits (1,0 mL) | 1,4 cm | 0,40 cm | / | 3 | / |
| 48 puits (0,8 mL) | 1,1 cm | 0,55 cm | / | / | 9 |
| 24 puits (0,5 mL) | 1,4 cm | 0,25 cm | / | 2 | / |
| 24 puits (0,8 mL) | 1, 4 cm | 0,35 cm | / | 6 | / |

| | | | | | |
|---|---|---|---|---|---|
| ^{∗}Pour les puits de plus grandes tailles, les broyats de gelée de Wharton viro-inactivée et lyophilisée présentent une fine pellicule sur les bords. La hauteur des broyats de gelée de Wharton viro-inactivée et lyophilisée sur les bords est donc plus grande. Cependant cette pellicule est très fine, de l'ordre de la dizaine de micromètre, et sur le reste du disque l'épaisseur est quasi constante. | | | | | |

### Résultats après réhydratation :

Trois essais de réhydratation sont effectués pour chacun des lyophilisats obtenus :

Un essai avec une faible hydratation (30 µL d'eau purifiée par mg de lyophilisat) donnant un aspect de gel visqueux, dont la cohésion est importante.

Un autre essai avec une hydratation plus liquide (32 µL d'eau purifiée par mg de lyophilisat) présentant une substance gélifiée.

Un troisième essai avec un excès d'eau (36 µL d'eau purifiée par mg de lyophilisat) afin d'identifier les tailles de fibres.

Le broyage avec un tamis de 3 mm puis un tamis de 1 mm permet d'avoir une texture de gel la plus intéressante.

Le volume de produit obtenu après réhydratation est environ égal à celui obtenu avant lyophilisation.

Le choix de la taille de la plaque dans laquelle sont répartis les aliquotes dépend de l'application visée (volume, épaisseur, diamètre du disque de broyat lyophilisé voulu).

### Conclusions :

La réhydratation des broyats de gelée de Wharton lyophilisée nécessite une agitation, celle-ci peut être effectuée à la spatule pour séparer efficacement les fibres et gagner en souplesse aisément.

L'aliquotage des broyats de gelée de Wharton est possible à effectuer sans problème dans des puits de plaques 96, 48, 24, 12 et 6 puits. La plaque choisie pour l'aliquotage va donc dépendre de l'application visée.

Le fait que le couvercle de la plaque recouvre ou non les puits lors de la lyophilisation ne semble pas avoir d'impact sur les produits.

Pour réhydrater les broyats de gelée de Wharton lyophilisée, entre 30 et 40 µL d'eau purifiée par mg de produit sont utilisées, selon l'application visée.

### Exemple 6 : Exemples de tamisages

Pour obtenir une meilleure homogénéité notamment des fibres de collagène, des passages au tamis vibrant sont effectués entre les différents broyages.

Par exemple un passage au tamis vibrant avec des dimensions progressives de 1,6 mm, 1 mm et 500 microns, est effectué lors des broyages pour éviter tout défaut d'homogénéité des fibres.

La gelée de Wharton récupérée dans le réceptacle après utilisation des tamis vibrant de 500 microns, permet d'obtenir un broyage avec des fibres homogènes.

Le broyat de gelée de Wharton est récupéré dans un flacon de 50 mL et envoyé en salle de lyophilisation où il est réparti dans des plaques de 24 puits.

Après répartition dans les puits, la gelée de Wharton est lyophilisée, une forme de disque est obtenue.

Lors de son utilisation, par exemple en bio-impression, le broyat de gelée de Wharton viro-inactivée et lyophilisée est réhydratée selon les volumes de réhydratation définis. Le gel est fluide, les tailles des fibres bien inférieures à 500 microns permettent de limiter les risques d'obstruction de l'aiguille de la bio-imprimante.

### Exemple 7 : Exemple de mise en forme du broyat de gelée de Wharton viro-inactivée et lyophilisée selon l'invention dans une bio-imprimante 3D

Un disque de composition selon l'invention obtenu aux exemples précédents est réhydraté dans une solution de PBS (1,4 mL pour 0,8 mL de disque).

Ce mélange (30% du volume final) est associé à un gel liquide par chauffage puis refroidi d'alginate (1% du volume final) et de gélatine (15% du volume final) puis passé dans l'aiguille de la bio-imprimante dont le diamètre interne est de 0,250 microns.

Le produit est ensuite utilisé dans la bio-imprimante pour générer par bio-impression un pourtour d'un parallélépipède dont la longueur d'arête est de 2 cm (base carrée du parallélépipède), et dont la hauteur est de 3 mm.

### Exemple 8 : Exemple d'un mode de réalisation de l'invention selon lequel un segment de gelée de Wharton est traité selon le procédé pour obtenir une composition utilisable dans le comblement osseux.

Un donneur proprement informé et consentant en accord avec les exigences de la Déclaration d'Helsinki offre en don le cordon ombilical issu d'un accouchement. Par exigence sanitaire relative aux dons de tissus et de cellules d'origine humaine, une qualification en amont du donneur est systématique. Cette qualification implique une recherche de virus HIV, hépatites B, C, HTLV et de la bactérie tréponème pâle responsable de la syphilis.

Le cordon ombilical est récupéré le plus tôt possible en salle d'accouchement. Il est avantageusement placé dans une boite stérile, comprenant une solution de NaCl à +4°C.

Au laboratoire, en salle stérile, la procédure suivante est appliquée :

Un segment de 20 cm à 50 cm de longueur est isolé par section du cordon ombilical.

Le cordon ombilical est rincé et hydraté dans des bains d'eau purifiée successifs, sous agitation douce, pendant 4 heures.

Les vaisseaux sanguins du segment de cordon ombilical sont identifiés et séparés du reste du segment afin de conserver uniquement la gelée de Wharton et la membrane amniotique qui l'entoure. La gelée de Wharton et la membrane amniotique sont utilisées dans la suite du procédé sous l'appellation générale de gelée de Wharton, car la quantité en poids de membrane est négligeable par rapport à la quantité en poids de gelée de Wharton.

La gelée de Wharton est congelée à sec à une température de -80°C.

Lors du traitement chimique, la gelée de Wharton est décongelée à l'air libre, à température ambiante, pendant une durée de 5 minutes. Cette étape de congélation, suivie d'une décongélation, assure une décellularisation importante du tissu biologique.

La gelée de Wharton subit une succession de bains assurant un traitement chimique de celle-ci. Ce traitement a pour objectif une désinfection de la gelée de Wharton, et tout particulièrement sa viro-inactivation. Une agitation linéaire douce du milieu liquide est appliquée lors de chaque bain afin d'assurer une pénétration homogène des solvants dans les tissus.

Dans un premier temps, la gelée de Wharton est déposée dans un bain d'eau purifiée à température ambiante pendant environ 3 heures. Cette étape assure tant la fin de la décongélation du tissu physiologique, qu'une étape de lyse cellulaire par pression osmotique.

Puis, la gelée de Wharton est transférée dans un bain décontaminant qui est composé d'éthanol 70 % v/v à température ambiante pendant environ 1 heure.

Un lavage est effectué dans de l'eau purifiée pendant environ 15 minutes à température ambiante pour retirer l'éthanol.

Afin d'assurer la seconde étape de traitement décontaminant, la gelée de Wharton est transférée dans un bain composé de peroxyde d'hydrogène à 30 % w/v à température ambiante pendant environ 15 minutes.

Puis, la gelée de Wharton est transférée dans un bain décontaminant qui est composé de peroxyde d'hydrogène à 3 % w/v à température ambiante pendant environ 1 heure. La gelée de Wharton obtenue est viro-inactivée.

L'action chimique appliquée à la gelée de Wharton viro-inactivée est ensuite neutralisée, dans un bain comportant de l'hydroxyde de sodium dilué autour d'un pH de 8,5. Le traitement du bain de neutralisation s'effectue à température ambiante pendant environ 15 minutes.

Afin d'assurer un rééquilibrage du pH et pour éliminer au mieux les résidus organiques se détachant du tissu d'intérêt, la gelée de Wharton viro-inactivée est transférée dans un bain de tampon physiologique (PBS), afin d'assurer son rééquilibrage physiologique. Le bain s'effectue à température ambiante pendant environ 15 minutes.

Enfin, la gelée de Wharton est transférée dans deux bains successifs à l'eau purifiée, à température ambiante, pendant au moins 15 minutes et jusqu'à environ 1 heure.

A cette étape du procédé, on peut considérer que la gelée de Wharton obtenue selon ce traitement chimique est une gelée de Wharton en grande partie désinfectée, notamment viro-inactivée, et décellularisée.

Suite à cette partie relative aux traitements chimiques, la gelée de Wharton viro-inactivée subit une étape de broyage.

La gelée de Wharton est insérée dans un broyeur vibrant à billes Retsch MM400, équipé avec un bol en oxyde de zirconium de 35 mL. La gelée de Wharton occupe un espace d'environ 1/3 du bol. Une bille en oxyde de zirconium d'un diamètre de 20 mm est ajoutée dans le bol avec la gelée de Wharton. Un broyage de 1 minute est effectué à une fréquence de 3 Hz. La bille d'un diamètre de 20 mm est récupérée, et 9 billes en oxyde de zirconium d'un diamètre de 10 mm sont ajoutées dans le bol. Un deuxième broyage de 1 minute est réalisé à une fréquence de 30 Hz. La substance obtenue est une substance gélifiée homogène. Cette substance est mise dans un flacon.

Pour pouvoir utiliser la gelée de Wharton dans le comblement osseux, de la poudre d'os spongieux minéralisé d'origine humaine est mélangée au broyat de gelée de Wharton viro-inactivée. Cette poudre d'os spongieux minéralisé est caractérisée en ce qu'elle a été préalablement viro-inactivée et lyophilisée. Cette poudre est également caractérisée par une granulométrie comprise entre 0,2 et 2,0 mm. Cette poudre est introduite et mélangée au broyat de gelée de Wharton viro-inactivée de telle sorte que la proportion en poids finale en poudre d'os spongieux minéralisé dans le produit soit de 50%.

A cette étape du procédé, la gelée de Wharton viro-inactivée et broyée, mélangée à de la poudre d'os spongieux minéralisé, est mise en forme dans un moule. Ce moule accompagne idéalement la composition tout au long de son cycle de vie, de l'étape de mise en forme jusqu'à la mise à disposition de l'utilisateur.

Le moule utilisé est plusieurs réceptacles en inox 316L (selon la norme américaine AISI : 316L ; selon la norme européenne EN 10027 : X2CrNiMo17-12-02 1.4404) de forme cylindrique dont le diamètre mesure 1,0 cm et dont la hauteur mesure 0,5 cm.

La gelée de Wharton viro-inactivée et broyée est mise en forme dans autant de moules que désirés et que la quantité de gelée de Wharton viro-inactivée et broyée permet. Une pipette de 1 mL est avantageusement utilisée pour mettre en forme la gelée de Wharton viro-inactivée et broyée dans les moules.

Suite à cette partie relative au broyage et à la mise en forme, la gelée de Wharton viro-inactivée et broyée subit une lyophilisation.

La gelée de Wharton viro-inactivée et broyée qui est contenue dans le moule est placée sur un plateau en inox.

L'ensemble est transféré dans un lyophilisateur, où une étape de congélation suivie d'une étape de lyophilisation sont effectuées selon les modalités suivantes :
- Congélation :
   - La première étape de congélation s'effectue à une température d'acclimatation choisie pour ne pas endommager l'intégrité structurelle, fonctionnelle et biologique de la gelée de Wharton viro-inactivée et broyée ;
   - la deuxième étape de congélation s'effectue à la température finale de congélation qui est inférieure à la température d'acclimatation ;
- Lyophilisation :
   - Une étape de lyophilisation primaire s'effectue par application d'un vide à environ 200 microbars et par application d'un profil de température ascendant ;
   - Une étape secondaire de lyophilisation s'effectue par application d'un vide à environ 50 microbars et par application d'un profil de température descendant.

Suite à cette étape de lyophilisation, le produit obtenu est un broyat de gelée de Wharton, désinfectée, notamment viro-inactivée, décellularisée et lyophilisée. De plus, le produit obtenu est défini par une forme de cylindre d'un diamètre de 1,0 cm et d'une hauteur de 0,3 cm, qui est conférée par le moule suite à l'étape de lyophilisation. Le produit obtenu est également caractérisé en ce qu'il comprend de la poudre d'os spongieux minéralisé.

Chacun des broyats de gelée de Wharton, viro-inactivée, décellularisée et lyophilisée ainsi formés est détaché aisément de son moule et repositionné dans le même moule et mis dans un conditionnement primaire qui est un sachet TYVEK^{®} en copolymère PE-PET.

D'autres moules de présentation et d'autres conditionnements primaires peuvent être utilisés selon les besoins des praticiens auxquels sont destinées les compositions selon l'invention.

Une dernière étape de stérilisation du broyat de gelée de Wharton, viro-inactivée, décellularisée et lyophilisée est effectuée par exposition de ce broyat à des rayonnements gamma à 25-32 kGrays. Tous les sachets comprenant les broyats obtenus à partir de la substance biologique initiale sont traités simultanément lors de cette étape de stérilisation par rayonnements gamma.

Le produit ainsi obtenu est utilisé comme composition pour le comblement d'une perte osseuse.

Il est intéressant de noter que les broyats de gelée de Wharton, viro-inactivée, décellularisée et lyophilisée ainsi formés peuvent également être directement utilisés par le praticien comme composition, par exemple, pour le comblement osseux, sans effectuer les étapes de conditionnement et de stérilisation. En effet, lesdits broyats se démoulent aisément et le praticien peut les appliquer directement sur le site biologique de l'intervention chirurgicale.

Quelques utilisations envisageables du produit obtenu selon le procédé sont par exemples le comblement osseux faisant suite à la perte osseuse consécutive à une trépanation, la perte osseuse consécutive à une exérèse d'un fragment d'os, la perte osseuse consécutive à une ostéoporose, la perte osseuse consécutive au forage d'une cavité dans un os.

Lorsque le produit est utilisé dans le traitement d'une perte osseuse consécutive à une trépanation réalisée par un praticien, la méthode consiste en l'introduction du produit obtenu selon le procédé dans l'orifice de l'os du crâne nécessitant le comblement. La forme du produit permet une adaptation aisée dans l'orifice percé par le praticien postérieurement à son acte de trépanation. En effet, le produit obtenu selon le procédé comprenant en outre de la poudre d'os, il possède une souplesse suffisante pour permettre au praticien de positionner correctement le produit dans l'orifice à combler, tout en possédant une rigidité suffisante pour ne pas se déformer ou se déplacer après son implantation. De plus, le produit obtenu selon le procédé comprenant en outre de la poudre d'os, il constitue un support privilégié pour que l'os du patient se reconstitue au sein de ce support. Le produit est réhydraté in situ.

### Exemple 9 : Mesure de la quantité d'acide hyaluronique dans une composition selon l'invention.

Des dosages d'acide hyaluronique par immunodosage ELISA ont été effectués sur des compositions selon l'invention.

Trois compositions obtenues selon le procédé décrit à l'exemple 1 (Composition la, Composition 1b, Composition 1c) ont été utilisées pour effectuer ces dosages. Les tissus biologiques d'origine, à savoir les cordons ombilicaux, sont issus de trois sources différentes.

Un extrait des trois compositions est découpé, pesé et dilué dans un volume d'eau distillé. La quantité d'acide hyaluronique présente dans les échantillons dilués sont mesurés par l'utilisation d'un kit pour le dosage ELISA de l'acide hyaluronique.

Les quantités mesurées sont ramenées par calcul aux teneurs en poids présentes dans les compositions Composition la, Composition 1b, Composition 1c avant dilution.

La Composition la a une teneur en poids d'acide hyaluronique de 8,0 mg/g de composition totale.

La Composition 1b a une teneur en poids d'acide hyaluronique de 11,3 mg/g de composition totale.

La Composition 1c a une teneur en poids d'acide hyaluronique de 9,7 mg/g de composition totale.

Une distribution homogène en acide hyaluronique dans les compositions est observée. Celles-ci comprennent environ 10 mg d'acide hyaluronique par gramme de composition, soit environ 1%.

### Exemple 10 : Mesure de la quantité de TGF-β1 dans une composition selon l'invention.

Des dosages de facteur de croissance, qui est le TGF (Transforming Growth Factor) de type béta 1, par immunodosage ELISA ont été effectués sur des compositions selon l'invention.

Trois compositions obtenues selon le procédé décrit à l'exemple 1 (Composition la, Composition 1b, Composition 1c) ont été utilisées pour effectuer ces dosages. Les tissus biologiques d'origine, à savoir les cordons ombilicaux, sont issus de trois sources différentes.

Un extrait des trois compositions est découpé, pesé et dilué dans un volume d'eau distillé. La quantité de TGF-β1 présente dans les échantillons dilués sont mesurés par l'utilisation d'un kit pour le dosage ELISA de TGF-β1.

Les quantités mesurées sont ramenées par calcul aux teneurs en poids présentes dans les compositions Composition la, Composition 1b, Composition 1c avant dilution.

La Composition la a une teneur en poids de TGF-β1 de 1,6 ng/g de composition totale.

La Composition 1b a une teneur en poids de TGF-β1 de 1,0 ng/g de composition totale.

La Composition 1c a une teneur en poids de TGF-β1 de 0,9 ng/g de composition totale.

La teneur en poids de TGF-β1 semble varier selon les échantillons. Il est globalement autour de 1 ng de TGF-β1 par gramme de composition.

### Exemple 11 : Mise en évidence de la dévitalisation d'une composition selon l'invention et de son application comme support de culture cellulaire.

L'efficacité de la dévitalisation d'une composition selon l'invention a été évaluée par l'utilisation d'un kit de viabilité.

Ce même kit a été utilisé pour visualiser la viabilité et l'attachement d'une culture cellulaire de cellules souches mésenchymateuses (abrégé MSC) sur une composition selon l'invention.

Matériel :
- Cellules MSC n°DP-MSC issues de cordon ombilical (3^{ème} passage d'ensemencement), mises en culture dans deux flasques T75 recouvertes de collagène.
- Milieu de culture avec sérum bovin et antibiotiques.
- Microscope à fluorescence.
- Kit Invitrogen « Live/Dead cell viability kit » (référence: 5578129, lot 16/214).
- 3 compositions selon l'invention, obtenues selon le procédé décrit à l'exemple 1. Les tissus biologiques d'origine de ces 3 compositions, à savoir les cordons ombilicaux, sont issus de trois sources différentes.
- Microplaques.

### Méthode :

Les compositions sont découpées en échantillons d'environ 5 mm de long et positionnés verticalement dans des puits de la microplaque, en les enroulant de sorte à former un anneau contre les parois du puit de la microplaque.

Un faible volume de milieu de culture est ajouté de sorte à réhydrater les échantillons.

Les cellules MSC sont ajoutées lentement aux échantillons réhydratés. Les cellules MSC sont ajoutées par introduction d'un volume de 33 µL du milieu d'ensemencement, ce qui correspond à environ 100 000 cellules.

Une série de puits est conservée sans ajout de MSC afin de visualiser l'efficacité de la dévitalisation des compositions.

Une série de puits sans échantillons de composition selon l'invention, mais avec ajout de MSC sert de contrôle positif.

Les microplaques ainsi obtenues sont incubées à 37°C, 5 % CO₂, en présence d'eau dans l'incubateur, pendant une heure afin de permettre aux MSC d'adhérer aux échantillons de compositions.

Le milieu de culture est ensuite ajouté dans les puits de sorte à les remplir en entièreté.

Les microplaques ainsi obtenues sont incubées à 37°C, 5 % CO₂, en présence d'eau dans l'incubateur. Le milieu de culture est changé à intervalle régulier (3 fois par semaine).

Après 15 jours de culture, le test de viabilité est réalisé à l'aide du kit selon les recommandations du fournisseur.

Chaque puit est observé en microscopie à fluorescence : les cellules viables apparaissent de couleur verte, les cellules mortes apparaissent en couleur rouge.

### Résultats :

Après 15 jours de culture, les parois des échantillons des trois compositions selon l'invention sont recouvertes d'une grande quantité de cellules émettant un rayonnement vert fluorescent sous observation en microscopie à fluorescence. Ces cellules sont également observées à l'intérieur des échantillons des trois compositions selon l'invention. Ces cellules émettant un rayonnement vert fluorescent sont des cellules viables.

Les MSC sont donc viables, ont adhéré aux échantillons des trois compositions selon l'invention, et ont colonisé tant la surface que l'intérieur des échantillons des trois compositions selon l'invention.

Les compositions selon l'invention sont donc des supports propices tant à la culture cellulaire qu'à la régénération cellulaire in vivo.

Après 10 jours de culture, l'observation en microscopie à fluorescence de la série de puits sans ajout de MSC révèle une faible quantité de cellules émettant un rayonnement rouge fluorescent. Ces cellules émettant un rayonnement rouge fluorescent sont des cellules mortes.

La dévitalisation des compositions selon l'invention est donc particulièrement efficace en ce que la faible quantité de cellules révélées par l'utilisation du kit « Live/Dead cell viability kit » sont uniquement des cellules mortes. Les compositions selon l'invention sont donc totalement dévitalisées.

## Revendications

1. Biomatériau consistant en un broyat homogène de gelée de Wharton viro-inactivée **caractérisé en ce que** les virus ont perdu leurs capacités pathogéniques et de réplication par une diminution de leur population de 4 log lors des titrages résiduels qui suivent une ou deux étapes chimiques indépendantes et **en ce que** le broyat ne comprend aucun fragment ou morceau dont les dimensions sont significativement supérieures à celles des éléments majoritaires du broyat et qu'il comprend l'intégralité des éléments constitutifs du tissu biologique initial qui est la gelée de Wharton.

2. Composition comprenant au moins un biomatériau selon la revendication 1.

3. Composition selon la revendication 2, **caractérisée en ce qu'**elle est lyophilisée.

4. Composition selon l'une quelconque des revendications 2 à 3, **caractérisée en ce qu'**elle comprend en outre un principe actif choisi dans le groupe constitué des antibiotiques, des antiseptiques, des antiviraux, des anticorps monoclonaux, des inhibiteurs semi-synthétiques, des métalloprotéases, des agents immunosuppresseurs, des anti-inflammatoires, des anti-cancéreux, des antifongiques, des anti-allergiques, des anesthésiques, des protéines immunoadhésives seuls ou en combinaisons.

5. Composition selon l'une quelconque des revendications 2 à 4, **caractérisée en ce qu'**elle comprend en outre un substitut osseux sous forme de poudre.

6. Composition selon la revendication 3, **caractérisée en ce qu'**elle est moulée.

7. Composition selon l'une quelconque des revendications 2 à 6, **caractérisée en ce qu'**elle est stérilisée.

8. Composition selon l'une quelconque des revendications 2 à 7, pour son utilisation dans le traitement des lésions de la surface oculaire, le traitement d'une lésion cutanée.

9. Composition selon l'une quelconque des revendications 2 à 7, pour son utlisation dans le comblement osseux.

10. Composition selon l'une quelconque des revendications 2 à 7, pour son utlisation dans le comblement d'une alvéole dentaire.

11. Procédé de préparation d'une composition selon l'une quelconque des revendications 2 à 7, **caractérisé en ce qu'**il comprend au moins les étapes suivantes :
a) on dispose d'un segment de gelée de Wharton dont la veine et les artères ont été ôtées ;
b) on effectue un traitement de viro-inactivation du segment de gelée de Wharton pour obtenir un segment de gelée de Wharton viro-inactivée ;
c) on effectue un broyage du segment de gelée de Wharton viro-inactivée pour obtenir un broyat homogène de gelée de Wharton viro-inactivée.

12. Procédé selon la revendication 11, **caractérisé en ce qu'**il comporte en outre une étape d), postérieure à l'étape c), définie comme suit :
d) on effectue une lyophilisation du broyat de gelée de Wharton viro-inactivée pour obtenir un broyat de gelée de Wharton viro-inactivée et lyophilisée.

13. Procédé selon les revendications 11 et 12, **caractérisé en ce que** le traitement de viro-inactivation comprend au moins deux étapes successives de traitement par un agent de viro-inactivation chimique.

14. Utilisation d'une composition selon les revendications 2 à 7, ou susceptible d'être obtenu selon le procédé selon les revendications 11 à 13 dans la bio-impression.

15. Méthode pour enrober des matériaux ou des cellules destinés à la bio-impression, **caractérisée en ce qu'**elle comprend les étapes suivantes :
- on introduit dans la composition selon les revendications 2 à 7, ou susceptible d'être obtenu selon le procédé selon les revendications 11 à 13, les biomatériaux ou les cellules destinés à être bio-imprimés ;
- on homogénéise le mélange précédemment obtenu afin d'obtenir une bioencre ;
- on introduit la bioencre dans la cartouche de la bio-imprimante.

## Patentansprüche

1. Biomaterial bestehend aus einem virusinaktivierten, homogenen zerkleinerten Wharton-Gelee-Stück, **dadurch gekennzeichnet, dass** die Viren Ihre Pathogenität und ihre Replikationsfähigkeit verloren haben durch eine Verringerung ihrer Zahl um 4 Logs bei der Resttitrierung nach einem oder zwei unabhängigen chemischen Schritten, und dass das zerkleinerte Stück kein Fragment oder kein Stückchen umfasst, dessen Abmessungen signifikant größer sind als diejenigen der mehrheitlichen Bestandteile des zerkleinerten Stückes, und es die Gesamtheit der Bausteine des ursprünglichen biologischen Gewebes umfasst, wobei dies das Wharton-Gelee ist.

2. Zusammensetzung umfassend wenigstens ein Biomaterial nach Anspruch 1.

3. Zusammensetzung nach Anspruch 2, **dadurch gekennzeichnet, dass** sie lyophilisiert ist.

4. Zusammensetzung nach einem der Ansprüche 2 bis 3, **dadurch gekennzeichnet, dass** sie außerdem einen Wirkstoff umfasst, der ausgewählt ist aus der Gruppe bestehend aus Antibiotika, Antiseptika, antiviralen Mitteln, monoklonalen Antikörpern, halbsynthetischen Inhibitoren, Metalloproteasen, Immunsuppressiva, entzündungshemmenden Mitteln, Krebsmitteln, Antimykotika, Antiallergika, Anästhetika, immunadhäsiven Proteinen alleine oder in Kombinationen.

5. Zusammensetzung nach einem der Ansprüche 2 bis 4, **dadurch gekennzeichnet, dass** sie außerdem ein Knochenersatzmaterial in Form eines Pulvers umfasst.

6. Zusammensetzung nach Anspruch 3, **dadurch gekennzeichnet, dass** sie geformt ist.

7. Zusammensetzung nach einem der Ansprüche 2 bis 6, **dadurch gekennzeichnet, dass** sie sterilisiert ist.

8. Zusammensetzung nach einem der Ansprüche 2 bis 7, zur Verwendung bei der Behandlung von Läsionen der Augenoberfläche oder der Behandlung einer Hautläsion.

9. Zusammensetzung nach einem der Ansprüche 2 bis 7, zur Verwendung bei der Knochenauffüllung.

10. Zusammensetzung nach einem der Ansprüche 2 bis 7, zur Verwendung bei der Auffüllung einer Zahnalveole.

11. Verfahren zur Herstellung einer Zusammensetzung nach einem der Ansprüche 2 bis 7, **dadurch gekennzeichnet, dass** es wenigstens die folgenden Schritte umfasst:
a) Bereitstellen eines Wharton-Gelee-Stückes, dessen Venen und Arterien entfernt worden sind;
b) Durchführen einer Virusinaktivierungsbehandlung des Wharton-Gelee-Stückes, um ein virusinaktiviertes Wharton-Gelee-Stück zu erhalten;
c) Zerkleinern des virusinaktivierten Wharton-Gelee-Stückes, um ein virusinaktiviertes, homogenes zerkleinertes Wharton-Gelee-Stück zu erhalten.

12. Verfahren nach Anspruch 11, **dadurch gekennzeichnet, dass** es ausserdem einen Schritt d) nach dem Schritt c) umfasst, der wie folgt definiert ist:
d) Lyophilisieren des virusinaktivierten zerkleinerten Wharton-Gelee-Stückes, um ein lyophilisiertes und virusinaktiviertes zerkleinertes Wharton-Gelee-Stück zu erhalten.

13. Verfahren nach einem der Ansprüche 11 und 12, **dadurch gekennzeichnet, dass** die Virusinaktivierungsbehandlung wenigstens zwei aufeinanderfolgende Behandlungsschritte mit einem chemischen virusinaktivierenden Mittel umfasst.

14. Verwendung einer Zusammensetzung nach einem der Ansprüche 2 bis 7, oder einer gemäß einem Verfahren nach einem der Ansprüche 11 bis 13 erhältlichen Zusammensetzung, beim Bioprinting.

15. Verfahren zum Beschichten von Materialien oder Zellen, die für das Bioprinting bestimmt sind, **dadurch gekennzeichnet, dass** es die folgenden Schritte umfasst:
- Einführen der einem Bioprinting zu unterziehenden Biomaterialien oder der einem Bioprinting zu unterziehenden Zellen in die Zusammensetzung nach einem der Ansprüche 2 bis 7 oder in die gemäß dem Verfahren nach einem der Ansprüche 11 bis 13 erhältlichen Zusammensetzung;
- Homogenisieren der zuvor erhaltenen Mischung, um eine biologische Tinte zu erhalten;
- Einführen der biologischen Tinte in die Kartusche des Bio-Druckers.

## Claims

1. Bio-material consisting of a homogenous virally inactivated ground material of Wharton's jelly **characterized in that** the viruses have lost their pathogenic and replication capabilities by decreasing their population by 4 log during residual titrations which follow one or two independent chemical steps and **in that** the ground material does not include any fragments or pieces whose dimensions are significantly larger than those of the majority of the ground material and that it comprises all of the constituent elements of the initial biological tissue which is the Wharton's jelly.

2. Composition comprising at least one biomaterial according to claim 1.

3. Composition according to claim 2, **characterized in that** it is freeze-dried.

4. Composition according to any of claims 2 to 3, **characterized in that** that it further comprises an active ingredient chosen from the group consisting of antibiotics, antiseptics, antivirals, monoclonal antibodies, semi-synthetic inhibitors, metalloproteases, immunosuppressive agents, anti-inflammatory drugs, anti-cancer agents, anti-fungal agents, anti-allergic agents, anesthetics, immunoadhesive proteins alone or in combination.

5. Composition according to any of claims 2 to 4, **characterized in that** that it further comprises a bone substitute in powder form.

6. Composition according to claim 3, **characterized in that** it is mould.

7. Composition according to any of claims 2 to 6, **characterized in that** it is sterilized.

8. Composition according to any of claims 2 to 7, for use in the treatment of lesions of the surface of the eye, treatment of a skin lesion.

9. Composition according to any of claims 2 to 7, for use in bone filling.

10. Composition according to any of claims 2 to 7, for use in dental socket filling.

11. Method for the preparation of a composition according to any of claims 2 to 7, **characterized in that** it comprises at least the following steps:
a) a segment of Wharton's jelly from which the vein and arteries have been removed is on hand;
b) a viral inactivation treatment of the Wharton's jelly segment is carried out in order to obtain a virally inactivated segment of Wharton's jelly;
c) grinding a segment of virally inactivated Wharton's jelly is carried out to obtain a homogeneous ground material of virally inactivated Wharton's jelly.

12. Method according to Claim 11, **characterized in that** it further comprises a step d), after step c), defined as follows:
d) freeze-drying of the virally inactivated ground material of Wharton's jelly is carried out to obtain freeze-dried and virally inactivated ground material of Wharton's jelly.

13. Method according to claims 11 and 12, **characterized in that** the viral inactivation treatment comprises at least two successive steps of treatment with a chemical viral inactivation agent.

14. Use of a composition according to claims 3 to 8, or obtainable by the method according to claims 11 to 13 in bioprinting.

15. Method for coating materials or cells designed for bioprinting, **characterized in that** it is comprised of the following steps:
- the biomaterials or cells that will be bio-printed are introduced into the composition according to claims 2 to 7, or obtainable by the method according to claims 11 to 13;
- the previously obtained mixture is homogenized in order to obtain a bio-ink.
- the bio-ink is introduced into the cartridge of the bio-printer.
